# EUROPEAN PATENT APPLICATION

(11) **EP 4 715 044 A1**
(43) Date of publication of application: **25.03.2026**
(21) Application number: 24838861.3
(22) Date of filing: 11.07.2024
(51) Int. Cl.: C12N 5/10, C12N 5/0783, A61K 35/17, A61K 35/12, A61P 35/00

(54) **CELL TREATMENT PRODUCT PREPARATION PROCESS AND USE THEREOF**

(30) Priority: 11.07.2023 CN 202310849738
(71) Applicant: Chongqing Precision Biotech Co., Ltd., Chongqing 400038 (CN)
(72) Inventor: WANG, Linling, Chongqing 400038 (CN); YANG, Zhi, Chongqing 400038 (CN); LI, Yunyan, Chongqing 400038 (CN); ZHANG, Qianzhen, Chongqing 400038 (CN); WANG, Meiling, Chongqing 400038 (CN); ZHU, Wei, Chongqing 400038 (CN); CHEN, Jun, Chongqing 400038 (CN); HUANG, Yi, Chongqing 400038 (CN); ZHENG, Jiao, Chongqing 400038 (CN); SHEN, Junjie, Chongqing 400038 (CN); HONG, Juan, Chongqing 400038 (CN)
(74) Representative: Monteiro Alves, Inês
(86) International application number: PCT/CN2024/104904
(87) International publication number: WO 2025/011609

(57) **Abstract**

The present application provides a method for preparing a cell population expressing a chimeric antigen receptor (CAR), and a composition, a culture medium or a kit applicable to the method. The cell population has reduced in vivo cytotoxicity.

## Description

### TECHNICAL FIELD

The present invention relates to the field of preparation of cell treatment products, and in particular, to a method of preparing a cell treatment product with reduced *in vivo* toxicity.

### BACKGROUND ART

Current CAR-T preparing technology includes four steps: (1) isolation of T cells, (2) activation of T cell, (3) CAR lentiviral transfection, and (4) of CAR-T cell expansion, with a preparation cycle of about 7-14 days. Herein, activation of T cells, CAR gene transfer, and *ex vivo* expansion are critical steps in the preparation of CAR-T cells, affecting an ultimate therapeutic effect of CAR-T cells. A good preparation process is fundamental to obtaining excellent CAR-T cells. Since resting T cells fail to achieve gene transfection, additional additives such as anti-CD3/CD28 antibodies or anti-CD3/CD28 antibody-coated magnetic beads are required for activation. The use of the additives not only increases the preparation costs, but also increases an operation step of removing the additives. However, the prolonged *ex vivo* expansion of the CAR-T cells not only results in an extended waiting period for the patients, but also leads to differentiation of CAR-T cells, thereby compromising the *in vivo* persistence of the infused CAR-T cells. Therefore, it is essential to conduct in-depth research and optimization on the critical steps in the CAR-T preparation, so as to, on one hand, reduce *ex vivo* differentiation of T cells and maintain better initial functional characteristics, thereby improving the therapeutic efficacy thereof, and on the other hand, shorten the preparation cycle, reduce the operation steps, and decrease the preparation costs, thereby accelerating the clinical and industrial translation thereof.

### SUMMARY

The present invention provides a method of rapidly preparing a cell population expressing a chimeric antigen receptor (CAR) without CD28 stimulation, and a composition and use for preparing the cell population expressing the CAR. The cell population prepared by the method of the present invention has better *in vivo* efficacy and safety.

Specifically, the present invention relates to the following.
1. A method of preparing a cell population expressing a chimeric antigen receptor (CAR), including:
   step (i): contacting the cell population with a CD3/TCR complex agonist;
   step (ii): contacting the cell population with a nucleic acid molecule encoding the CAR, thereby providing cells containing the nucleic acid molecule, and
   step (iii): culturing the cell population for a period of time, and harvesting the cell population.
2. The method according to item 1, wherein the cell population is not in contact with a CD28 agonist in step (i), steps (i) and (ii), steps (i), (ii), and (iii), or throughout an entire preparation process.
3. The method according to item 1 or 2, wherein the cell population is not in contact with an agonist for cell surface co-stimulatory molecules in step (i), steps (i) and (ii), steps (i), (ii), and (iii), or throughout an entire preparation process, wherein the co-stimulatory molecules include: CD28, ICOS, CD27, HVEM, LIGHT, CD40, 4-1BB, OX40, DR3, GITR, CD30, TIM1, CD2, and CD226. In some embodiments, the agonist for the cell surface co-stimulatory molecules is an antibody against the surface co-stimulatory molecules.
4. The method according to any one of items 1-3, wherein the CD3/TCR complex agonist includes an anti-CD3 antibody. In some embodiments, the anti-CD3 antibody comprises a domain specifically binding to a CD3ε chain. In some embodiments, the anti-CD3 antibody is a monoclonal antibody targeting CD3 or an antigen-binding fragment thereof, a bispecific or multispecific antibody targeting CD3 and other antigenic molecules or an antigen-binding fragment thereof. In some embodiments, the anti-CD3 antibody is an IgG subtype antibody, e.g., a humanized or human IgG subtype antibody, or an antigen-binding fragment thereof.
5. The method according to any one of items 1-4, wherein the cell population is in contact with an immune cell-activating cytokine in step i), steps i) and ii), steps i), ii), and iii), or throughout an entire preparation process, wherein the immune cell-activating cytokine excludes IL-2.
6. The method according to item 5, wherein the immune cell-activating cytokine comprises one or more selected from the group consisting of IL-7, IL-15, IL-21, IL-12, IL-18, and IL-6.
7. The method according to item 6, wherein the cytokine comprises a combination of IL-7 and IL-21.
8. The method according to item 6 or 7, wherein a concentration of the IL-7, when in contact with the cell population, is 2.5-10 ng/ml, such as 3 ng/ml, 3.5 ng/ml, 4 ng/ml, 4.5 ng/ml, 5 ng/ml, 5.1 ng/ml, 5.2 ng/ml, 5.3 ng/ml, 5.4 ng/ml, 5.5 ng/ml, 5.6 ng/ml, 5.7 ng/ml, 5.8 ng/ml, 5.9 ng/ml, 6 ng/ml, 6.5 ng/ml, 7 ng/ml, 7.5 ng/ml, 8 ng/ml, 8.5 ng/ml, 9 ng/ml, 9.5 ng/ml, or any value in the range of 2.5-10 ng/ml.
9. The method according to any one of items 6-8, wherein a concentration of the IL-21 when in contact with the cell population is 12.5-50 ng/ml, such as 13 ng/ml, 13.5 ng/ml, 14 ng/ml, 14.5 ng/ml, 15 ng/ml, 15.5 ng/ml, 16 ng/ml, 16.5 ng/ml, 17 ng/ml, 17.5 ng/ml, 18 ng/ml, 18.5 ng/ml, 19 ng/ml, 19.5 ng/ml, 20 ng/ml, 20.5 ng/ml, 21 ng/ml, 21.5 ng/ml, 22 ng/ml, 22.5 ng/ml, 23 ng/ml, 23.5 ng/ml, 24 ng/ml, 24.5 ng/ml, 25 ng/ml, 25.5 ng/ml, 26 ng/ml, 26.5 ng/ml, 27 ng/ml, 27.5 ng/ml, 28 ng/ml, 28.5 ng/ml, 29 ng/ml, 29.5 ng/ml, 30 ng/ml, 30.5 ng/ml, 31 ng/ml, 31.5 ng/ml, 32 ng/ml, 32.5 ng/ml, 33 ng/ml, 33.5 ng/ml, 34 ng/ml, 34.5 ng/ml, 35 ng/ml, 35.5 ng/ml, 36 ng/ml, 36.5 ng/ml, 37 ng/ml, 37.5 ng/ml, 38 ng/ml, 38.5 ng/ml, 39 ng/ml, 39.5 ng/ml, 40 ng/ml, 40.5 ng/ml, 41 ng/ml, 41.5 ng/ml, 42 ng/ml, 42.5 ng/ml, 43 ng/ml, 43.5 ng/ml, 44 ng/ml, 44.5 ng/ml, 45 ng/ml, 45.5 ng/ml, 46 ng/ml, 46.5 ng/ml, 47 ng/ml, 47.5 ng/ml, 48 ng/ml, 48.5 ng/ml, 49 ng/ml, 49.5 ng/ml, or any value in the range of 12.5-50 ng/ml, and the concentration may change gradually over the time of contact, for instance, decreasing.
10. The method according to item 4, wherein a concentration of the anti-CD3 antibody in contact with the cell population in step i) is ≥ 25 ng/ml, for example, 25-200 ng/ml or any value therein, such as 26 ng/ml, 27 ng/ml, 28 ng/ml, 29 ng/ml, 30 ng/ml, 31 ng/ml, 32 ng/ml, 33 ng/ml, 34 ng/ml, 35 ng/ml, 36 ng/ml, 37 ng/ml, 38 ng/ml, 39 ng/ml, 40 ng/ml, 41 ng/ml, 42 ng/ml, 43 ng/ml, 44 ng/ml, 45 ng/ml, 46 ng/ml, 47 ng/ml, 48 ng/ml, 49 ng/ml, 50 ng/ml, 51 ng/ml, 52 ng/ml, 53 ng/ml, 54 ng/ml, 55 ng/ml, 56 ng/ml, 57 ng/ml, 58 ng/ml, 59 ng/ml, 60 ng/ml, 61 ng/ml, 62 ng/ml, 63 ng/ml, 64 ng/ml, 65 ng/ml, 66 ng/ml, 67 ng/ml, 68 ng/ml, 69 ng/ml, 70 ng/ml, 71 ng/ml, 72 ng/ml, 73 ng/ml, 74 ng/ml, 75 ng/ml, 76 ng/ml, 77 ng/ml, 78 ng/ml, 79 ng/ml, 80 ng/ml, 81 ng/ml, 82 ng/ml, 83 ng/ml, 84 ng/ml, 85 ng/ml, 86 ng/ml, 87 ng/ml, 88 ng/ml, 89 ng/ml, 90 ng/ml, 91 ng/ml, 92 ng/ml, 93 ng/ml, 94 ng/ml, 95 ng/ml, 96 ng/ml, 97 ng/ml, 98 ng/ml, 99 ng/ml, 100 ng/ml, 101 ng/ml, 102 ng/ml, 103 ng/ml, 104 ng/ml, 105 ng/ml, 106 ng/ml, 107 ng/ml, 108 ng/ml, 109 ng/ml, 110 ng/ml, 111 ng/ml, 112 ng/ml, 113 ng/ml, 114 ng/ml, 115 ng/ml, 116 ng/ml, 117 ng/ml, 118 ng/ml, 119 ng/ml, 120 ng/ml, 121 ng/ml, 122 ng/ml, 123 ng/ml, 124 ng/ml, 125 ng/ml, 126 ng/ml, 127 ng/ml, 128 ng/ml, 129 ng/ml, 130 ng/ml, 131 ng/ml, 132 ng/ml, 133 ng/ml, 134 ng/ml, 135 ng/ml, 136 ng/ml, 137 ng/ml, 138 ng/ml, 139 ng/ml, 140 ng/ml, 141 ng/ml, 142 ng/ml, 143 ng/ml, 144 ng/ml, 145 ng/ml, 146 ng/ml, 147 ng/ml, 148 ng/ml, 149 ng/ml, 150 ng/ml, 151 ng/ml, 152 ng/ml, 153 ng/ml, 154 ng/ml, 155 ng/ml, 156 ng/ml, 157 ng/ml, 158 ng/ml, 159 ng/ml, 160 ng/ml, 161 ng/ml, 162 ng/ml, 163 ng/ml, 164 ng/ml, 165 ng/ml, 166 ng/ml, 167 ng/ml, 168 ng/ml, 169 ng/ml, 170 ng/ml, 171 ng/ml, 172 ng/ml, 173 ng/ml, 174 ng/ml, 175 ng/ml, 176 ng/ml, 177 ng/ml, 178 ng/ml, 179 ng/ml, 180 ng/ml, 181 ng/ml, 182 ng/ml, 183 ng/ml, 184 ng/ml, 185 ng/ml, 186 ng/ml, 187 ng/ml, 188 ng/ml, 189 ng/ml, 190 ng/ml, 191 ng/ml, 192 ng/ml, 193 ng/ml, 194 ng/ml, 195 ng/ml, 196 ng/ml, 197 ng/ml, 198 ng/ml, or 199 ng/ml.
11. The method according to any one of items 1-3, wherein the cell population is in contact with an immune cell-activating cytokine in step i), steps i) and ii), steps i), ii), and iii), or throughout an entire preparation process, wherein the immune cell-activating cytokine includes IL-2.
12. The method according to item 11, wherein a concentration of the IL-2 is 100 IU/ml-500 IU/ml, 150 IU/ml-450 IU/ml, 200 IU/ml-400 IU/ml, or 250 IU/ml-350 IU/ml.
13. The method according to any one of items 1-12, wherein a duration from start of step ii) to harvest of the cell population is ≤ 72 hours or ≤ 48 hours.
14. The method according to any one of items 1-13, wherein a duration from start of step ii) to harvest of the cell population is ≥ 6 hours. In some embodiments, in the method, the duration from the start of step ii) to the harvest of the cell population is ≥ 6 hours and ≤ 72 hours, such as 7 hours, 8 hours, 9 hours, 10 hours, 11 hours, 12 hours, 13 hours, 14 hours, 15 hours, 16 hours, 17 hours, 18 hours, 19 hours, 20 hours, 21 hours, 22 hours, 23 hours, 24 hours, 25 hours, 26 hours, 27 hours, 28 hours, 29 hours, 30 hours, 31 hours, 32 hours, 33 hours, 34 hours, 35 hours, 36 hours, 37 hours, 38 hours, 39 hours, 40 hours, 41 hours, 42 hours, 43 hours, 44 hours, 45 hours, 46 hours, 47 hours, 48 hours, 49 hours, 50 hours, 51 hours, 52 hours, 53 hours, 54 hours, 55 hours, 56 hours, 57 hours, 58 hours, 59 hours, 60 hours, 61 hours, 62 hours, 63 hours, 64 hours, 65 hours, 66 hours, 67 hours, 68 hours, 69 hours, 70 hours, or 71 hours, that is, harvesting the cell population at any time point from 6 hours to 72 hours after the start of step ii) is encompassed within the scope of the present invention.
15. The method according to any one of items 1-14, wherein a duration from step i) until start of step ii) is 12 to 24 hours, and a duration from the start of step ii) to harvest of the cell population is 14-28 hours.
16. The method according to any one of items 1-15, wherein a duration from start of step i) to harvest of the cell population is 1 to 4 days, such as 2 days.
17. The method according to any one of items 1-16, wherein a duration from start of step i) to harvest of the cell population is 1 to 2 days.
18. The method according to any one of items 1-17, wherein the cell population is immune cells, such as macrophages, NK cells, T cells, B cells, and dendritic cells. In some embodiments, the cell population is T cells or NK cells.
19. The method according to item 18, wherein the T cells are derived from PBMCs. In some embodiments, the cell population is peripheral blood mononuclear cells (PBMCs). In some embodiments, the cell population harvested in step (iii) is immune cells expressing CAR, such as CAR-T or CAR-NK.
20. A composition for preparing a cell population expressing a chimeric antigen receptor (CAR), comprising a CD3/TCR complex agonist and an immune cell-activating cytokine, wherein the immune cell-activating cytokine excludes or comprises IL-2. In embodiments comprising IL-2, a concentration of the IL-2 is 100 IU/ml-500 IU/ml, 150 IU/ml-450 IU/ml, 200 IU/ml-400 IU/ml, or 250 IU/ml-350 IU/ml.
21. The composition according to item 20, wherein the CD3/TCR complex agonist comprises an anti-CD3 antibody. In some embodiments, the anti-CD3 antibody comprises a domain specifically binding to a CD3ε chain. In some embodiments, the anti-CD3 antibody is IgG, which comprises a domain specifically binding to a CD3ε chain. In some embodiments, the anti-CD3 antibody is an IgG-type humanized antibody, which comprises a domain specifically binding to a CD3ε chain.
22. The composition according to item 21, wherein a concentration of the anti-CD3 antibody is ≥ 25 ng/ml, for example, 25-200 ng/ml or any value therein, such as 26 ng/ml, 27 ng/ml, 28 ng/ml, 29 ng/ml, 30 ng/ml, 31 ng/ml, 32 ng/ml, 33 ng/ml, 34 ng/ml, 35 ng/ml, 36 ng/ml, 37 ng/ml, 38 ng/ml, 39 ng/ml, 40 ng/ml, 41 ng/ml, 42 ng/ml, 43 ng/ml, 44 ng/ml, 45 ng/ml, 46 ng/ml, 47 ng/ml, 48 ng/ml, 49 ng/ml, 50 ng/ml, 51 ng/ml, 52 ng/ml, 53 ng/ml, 54 ng/ml, 55 ng/ml, 56 ng/ml, 57 ng/ml, 58 ng/ml, 59 ng/ml, 60 ng/ml, 61 ng/ml, 62 ng/ml, 63 ng/ml, 64 ng/ml, 65 ng/ml, 66 ng/ml, 67 ng/ml, 68 ng/ml, 69 ng/ml, 70 ng/ml, 71 ng/ml, 72 ng/ml, 73 ng/ml, 74 ng/ml, 75 ng/ml, 76 ng/ml, 77 ng/ml, 78 ng/ml, 79 ng/ml, 80 ng/ml, 81 ng/ml, 82 ng/ml, 83 ng/ml, 84 ng/ml, 85 ng/ml, 86 ng/ml, 87 ng/ml, 88 ng/ml, 89 ng/ml, 90 ng/ml, 91 ng/ml, 92 ng/ml, 93 ng/ml, 94 ng/ml, 95 ng/ml, 96 ng/ml, 97 ng/ml, 98 ng/ml, 99 ng/ml, 100 ng/ml, 101 ng/ml, 102 ng/ml, 103 ng/ml, 104 ng/ml, 105 ng/ml, 106 ng/ml, 107 ng/ml, 108 ng/ml, 109 ng/ml, 110 ng/ml, 111 ng/ml, 112 ng/ml, 113 ng/ml, 114 ng/ml, 115 ng/ml, 116 ng/ml, 117 ng/ml, 118 ng/ml, 119 ng/ml, 120 ng/ml, 121 ng/ml, 122 ng/ml, 123 ng/ml, 124 ng/ml, 125 ng/ml, 126 ng/ml, 127 ng/ml, 128 ng/ml, 129 ng/ml, 130 ng/ml, 131 ng/ml, 132 ng/ml, 133 ng/ml, 134 ng/ml, 135 ng/ml, 136 ng/ml, 137 ng/ml, 138 ng/ml, 139 ng/ml, 140 ng/ml, 141 ng/ml, 142 ng/ml, 143 ng/ml, 144 ng/ml, 145 ng/ml, 146 ng/ml, 147 ng/ml, 148 ng/ml, 149 ng/ml, 150 ng/ml, 151 ng/ml, 152 ng/ml, 153 ng/ml, 154 ng/ml, 155 ng/ml, 156 ng/ml, 157 ng/ml, 158 ng/ml, 159 ng/ml, 160 ng/ml, 161 ng/ml, 162 ng/ml, 163 ng/ml, 164 ng/ml, 165 ng/ml, 166 ng/ml, 167 ng/ml, 168 ng/ml, 169 ng/ml, 170 ng/ml, 171 ng/ml, 172 ng/ml, 173 ng/ml, 174 ng/ml, 175 ng/ml, 176 ng/ml, 177 ng/ml, 178 ng/ml, 179 ng/ml, 180 ng/ml, 181 ng/ml, 182 ng/ml, 183 ng/ml, 184 ng/ml, 185 ng/ml, 186 ng/ml, 187 ng/ml, 188 ng/ml, 189 ng/ml, 190 ng/ml, 191 ng/ml, 192 ng/ml, 193 ng/ml, 194 ng/ml, 195 ng/ml, 196 ng/ml, 197 ng/ml, 198 ng/ml, or 199 ng/ml. In some embodiments, the anti-CD3 antibody is 50 ng/ml.
23. The composition according to any one of items 20-22, wherein the immune cell-activating cytokine comprises one or more selected from the group consisting of IL-7, IL-15, IL-21, IL-12, IL-18, and IL-6.
24. The composition according to item 23, wherein the immune cell-activating cytokine comprises a combination of IL7 and IL21.
25. The composition according to item 24, wherein a concentration of the IL-7 is 2.5-10 ng/ml, such as 3 ng/ml, 3.5 ng/ml, 4 ng/ml, 4.5 ng/ml, 5 ng/ml, 5.1 ng/ml, 5.2 ng/ml, 5.3 ng/ml, 5.4 ng/ml, 5.5 ng/ml, 5.6 ng/ml, 5.7 ng/ml, 5.8 ng/ml, 5.9 ng/ml, 6 ng/ml, 6.5 ng/ml, 7 ng/ml, 7.5 ng/ml, 8 ng/ml, 8.5 ng/ml, 9 ng/ml, 9.5 ng/ml, or any value in the range of 2.5-10 ng/ml.
26. The composition according to item 24 or 25, wherein a concentration of the IL-21 is 12.5-50 ng/ml, such as 13 ng/ml, 13.5 ng/ml, 14 ng/ml, 14.5 ng/ml, 15 ng/ml, 15.5 ng/ml, 16 ng/ml, 16.5 ng/ml, 17 ng/ml, 17.5 ng/ml, 18 ng/ml, 18.5 ng/ml, 19 ng/ml, 19.5 ng/ml, 20 ng/ml, 20.5 ng/ml, 21 ng/ml, 21.5 ng/ml, 22 ng/ml, 22.5 ng/ml, 23 ng/ml, 23.5 ng/ml, 24 ng/ml, 24.5 ng/ml, 25 ng/ml, 25.5 ng/ml, 26 ng/ml, 26.5 ng/ml, 27 ng/ml, 27.5 ng/ml, 28 ng/ml, 28.5 ng/ml, 29 ng/ml, 29.5 ng/ml, 30 ng/ml, 30.5 ng/ml, 31 ng/ml, 31.5 ng/ml, 32 ng/ml, 32.5 ng/ml, 33 ng/ml, 33.5 ng/ml, 34 ng/ml, 34.5 ng/ml, 35 ng/ml, 35.5 ng/ml, 36 ng/ml, 36.5 ng/ml, 37 ng/ml, 37.5 ng/ml, 38 ng/ml, 38.5 ng/ml, 39 ng/ml, 39.5 ng/ml, 40 ng/ml, 40.5 ng/ml, 41 ng/ml, 41.5 ng/ml, 42 ng/ml, 42.5 ng/ml, 43 ng/ml, 43.5 ng/ml, 44 ng/ml, 44.5 ng/ml, 45 ng/ml, 45.5 ng/ml, 46 ng/ml, 46.5 ng/ml, 47 ng/ml, 47.5 ng/ml, 48 ng/ml, 48.5 ng/ml, 49 ng/ml, 49.5 ng/ml, or any value in the range of 12.5-50 ng/ml.
27. The composition according to any one of items 20 to 26, excluding a CD28 agonist. In some embodiments, the composition excludes any agonist for cell surface co-stimulatory molecules, wherein the co-stimulatory molecules include: CD28, ICOS, CD27, HVEM, LIGHT, CD40, 4-1BB, OX40, DR3, GITR, CD30, TIM1, CD2, and CD226.
28. A kit, including the composition according to any one of items 20-27, and excluding IL-2 and CD28 agonist.
29. Use of the composition according to any one of items 20-27, or the kit according to item 28 for preparing a cell population expressing a chimeric antigen receptor (CAR).
30. The use according to item 29, wherein the cell population comprises T cells. In some embodiments, the cell population expressing the chimeric antigen receptor (CAR) comprises CAR-T cells.
31. A cell culture medium for preparing a cell population expressing a chimeric antigen receptor (CAR), comprising a CD3/TCR complex agonist and an immune cell-activating cytokine, wherein the immune cell-activating cytokine excludes IL-2. In some embodiments, the anti-CD3 antibody comprises a domain specifically binding to a CD3ε chain. In some embodiments, the anti-CD3 antibody is a humanized or human antibody of IgG subtype or isotype .
32. The cell culture medium according to item 31, wherein the CD3/TCR complex agonist comprises an anti-CD3 antibody.

### BRIEF DESCRIPTION OF THE DRAWINGS

FIG. 1A shows CAR expression positive rates of CAR-T cells prepared by different preparation processes.
FIG. 1B shows viability detection of CAR-T cells from different preparation processes after cryopreservation and recovery.
FIG. 1C shows the number of rounds of target cell stimulation that CAR-T cells from different preparation solutions can withstand at an effector-to-target ratio of 1:2.
FIG. 1D shows proliferation potential of CAR-T cells from different preparation solutions after multiple rounds of stimulation at the effector-to-target ratio of 1:2.
FIG. 2A shows CAR-T activation detection after varying activation durations.
FIG. 2B shows CAR expression detection after varying activation durations.
FIG. 2C shows CAR expression detection of cells harvested at varying time after viral transduction.
FIG. 3A shows *in vivo* persistence detection of CAR-T cells from two preparation processes.
FIG. 3B shows *in vivo* efficacy of CD19-targeting CAR-T cells from two preparation processes.
FIG. 3C shows *in vivo* efficacy of CAR-T cells targeting CD19/CD22 or CEA from two preparation processes.
FIG. 4A shows viability of CAR-T cells prepared from different stimulation and activation solutions.
FIG. 4B shows phenotype of CAR-T prepared from different stimulation and activation solutions upon tumor cell stimulation.
FIG. 4C shows *in vivo* efficacy validation of CAR-T prepared from different stimulation and activation solutions.
FIG. 4D shows *in vivo* safety validation of CAR-T prepared from different stimulation and activation solutions.
FIG. 5 shows CAR expression detection of CAR-T cells under conditions of different cytokine combination solutions.
FIG. 6A shows viability detection of CAR-T cells after cryopreservation and recovery under conditions of different cytokine combination solutions.
FIG. 6B shows CAR positive rate detection of CAR-T cells under conditions of different cytokine combination solution.
FIG. 7A shows *in vivo* efficacy validation of CAR-T cells under conditions of different cytokine combination solutions.
FIG. 7B shows efficacy results of CAR-T cells targeting CD19/CD22 prepared by different processes.
FIG. 7C shows secretion results of IL-2 from different processes.
FIG. 7D shows secretion results of IFN-γ from different processes.
FIG. 7E shows secretion results of TNF-α from different processes.
FIG. 7F shows efficacy results of CD19-targeting CAR-T cells prepared by different processes.
FIG. 7G shows cytokine secretion after tumor cell killing by CD19-targeting CAR-T cells prepared by different processes.
FIG. 8A shows cell activation percentages in solutions with different anti-CD3 antibody concentrations.
FIG. 8B shows CAR positive rates at different anti-CD3 antibody concentrations.
FIG. 8C shows multiple rounds of stimulation for CAR-T cells, prepared in solutions with different anti-CD3 antibody concentrations, at an effector-to-target ratio of 1:1.
FIG. 8D shows efficacy of CAR-T prepared in P-CAR culture solutions with different anti-CD3 antibody concentrations.
FIG. 9A shows CAR positive rates at different concentrations of cytokine IL7/IL-21.
FIG. 9B shows multiple rounds of stimulation for CAR-T cells at different cytokine concentrations at an effector-to-target ratio of 1:1.
FIG. 9C shows comparison of tumor cell killing by different short-duration preparation solutions.
FIG. 9D shows comparison of IL-2 cytokine secretion capacity of CAR-T cells prepared from different short-duration preparation solutions upon tumor cell activation.
FIG. 10A shows killing validation of a P-CAR solution with IL-2 as a sole cytokine component.
FIG. 10B shows killing validation of a P-CAR solution with IL-7 as a sole cytokine component.
FIG. 10C shows killing validation of a P-CAR solution with IL-21 as a sole cytokine component.
FIG. 11A shows IL-2 secretion validation of the P-CAR solution with IL-2 as the sole cytokine component.
FIG. 11B shows IL-2 secretion validation of the P-CAR solution with IL-7 as the sole cytokine component.
FIG. 11C shows IL-2 secretion validation of the P-CAR solution with IL-21 as the sole cytokine component.

### DETAILED DESCRIPTION OF EMBODIMENTS

Interleukin-2 (IL-2) was first reported in 1976 and has been considered for decades as a key molecule for promoting T cell proliferation and differentiation, thus also being known as a T cell growth factor. However, in recent years, researchers have increasingly discovered that cell products cultured using IL-2 may offer stronger toxicity *in vivo,* and can induce CD8+ T cell exhaustion.

The present invention provides a method of preparing engineered immune cells. The method eliminates the use of a CD28 agonist, making it safer. Further, the method of the present invention may eliminate the use of IL-2, and successfully achieve zero usage of IL-2 by selecting a cell-stimulating factor. Moreover, the preparing method features rapid preparation, that is, the engineered immune cells, such as T cells, can be obtained within 1-4 days. In some embodiments, the method also incorporates a combination of specific cytokines for cell culture in a preparation process. In some embodiments, the method further enhances a tumoricidal effect and *in vivo* safety of the cell population by optimizing the duration of each preparation step. In addition, the present invention further provides a composition, a culture medium, and a kit suitable for use in the method.

### Definitions

Unless otherwise defined, all of the technical and scientific terms used herein have the meanings generally understood by those skilled in the art. The following references provide those skilled in the art with general definitions of many terms used in the present invention: Singleton et al., *Dictionary of Microbiology and Molecular Biology* (2nd ed. 1994); *The Cambridge Dictionary of Science and Technology* (Walker ed., 1988); *The Glossary of Genetics,* 5th Ed., R. Rieger et al (eds.), Springer Verlag (1991); and Hale & Marham, *The Harper Collins Dictionary of Biology* (1991). As used herein, the following terms have the meanings ascribed to them unless otherwise indicated.

As used herein, "CD3/TCR complex agonist" includes any antibody, ligand, or any small molecule compound that can bind to any subunit(s) of CD3 or TCR, and it falls within the scope of the term "CD3/TCR complex agonist" provided that its binding to the CD3/TCR complex can phosphorylate tyrosine residues in the ITAM (immunoreceptor tyrosine activation motif) of CD3, thereby causing downstream pathway activation. In some embodiments, the "CD3/TCR complex agonist" binds to any one or more chains of CD3. In some embodiments, the "CD3/TCR complex agonist" binds to one, two, or more of ε, y, and δ chains of CD3. In some embodiments, the "CD3/TCR complex agonist" is one or more selected from the group consisting of an antibody, a small molecule, and a ligand (e.g., a naturally occurring ligand, a recombinant ligand, or a chimeric ligand). In some embodiments, the "CD3/TCR complex agonist" is an anti-CD3 monoclonal or polyclonal antibody. In some embodiments, the "CD3/TCR complex agonist" is an anti-CD3 antibody, i.e., an anti-CD3 monoclonal or polyclonal antibody. In some embodiments, the monoclonal or polyclonal antibody comprises an antigen-binding fragment specifically binding to ε chain, γ chain, and/or δ chain of CD3. In some embodiments, the anti-CD3 antibody is an anti-human CD3 antibody. In some embodiments, the anti-CD3 antibody is a humanized antibody. In some embodiments, the anti-CD3 antibody is a humanized antibody targeting human CD3ε chain.

As used herein, the term "antibody" has its broadest meaning including full-length monoclonal antibodies, polyclonal antibodies, and, unless otherwise specified or contradicted to context, antigen-binding fragments, antibody variants, and multispecific molecules thereof, provided that they exhibit desired biological activity (i.e., activity to bind to an antigen or ligand thereof). In general, the term "full-length antibody" is a glycoprotein comprising at least two heavy chains and two light chains interconnected by disulfide bonds. The term antibody refers to an immunoglobulin molecule and an immunologically active fragment of immunoglobulin molecule, namely molecules containing an antigen-binding site. The immunoglobulin molecule can be of any type (e.g., IgG, IgE, IgM, IgD, IgA, and IgY), class (e.g., IgG1, IgG2, IgG3, IgG4, IgA1, and IgA2), or subclass. Thus, an "antibody" can also be a single variable domain (VHH) antibody, also referred to as a heavy-chain-only antibody (HcAb), which is devoid of light chains and may be naturally produced by camels or sharks. An antigen-binding portion of an HcAb is composed of a VHH fragment. As used herein, the term "antigen-binding fragment" refers to one or more fragments of a full length antibody that retain the ability to specifically bind to an antigen (e.g., CD3), which includes the antigen-binding site or variable region of an intact antibody, which in some cases excludes a constant region (i.e., CH2, CH3, and/or CH4, depending on an isotype of the antibody). antibody fragments include such as Fab, Fab', Fab'-SH, F(ab')2, and Fv fragments; polypeptides consisting of an contiguous sequence of amino acid residues derived from the antibody; and diabodies, and the like. The term "antigen-binding fragment" includes, but is not limited to, (1) a single chain Fv (scFv) molecule, (2) a single chain polypeptide including only one light chain variable domain, or a fragment thereof including three CDRs of a light chain variable domain, without a heavy chain molecule, and (3) a single chain polypeptide including only one heavy chain variable region, or a fragment thereof including three CDRs of a heavy chain variable region, without a light chain molecule; and a multispecific or multivalent structure formed from antibody fragments.

As used herein, "agonist for cell surface co-stimulatory molecules" is a compound or composition stimulating CD28, ICOS, CD27, HVEM, LIGHT, CD40, 4-1BB, OX40, DR3, GITR, CD30, TIM1, CD2, CD226, or any combination thereof. In some embodiments, the "agonist for cell surface co-stimulatory molecules" is a CD28 agonist. In some embodiments, the "agonist for cell surface co-stimulatory molecules" includes any antibody, ligand or any small molecule compound that can bind to CD28, ICOS, CD27, HVEM, LIGHT, CD40, 4-1BB, OX40, DR3, GITR, CD30, TIM1, CD2, and/or CD226, and can produce a co-stimulatory signal of the CD3/TCR complex. Likewise, the "CD28 agonist" refers to any antibody, ligand, or any small molecule compound that can bind to CD28 and generate a co-stimulatory signal of the CD3/TCR complex through binding to CD28.

As used herein, "cytokines" can be used to refer to a class of any small-molecule proteins with broad biological activity that are synthesized and secreted by immune cells (such as monocytes, macrophages, T cells, B cells, and NK cells) and certain non-immune cells (such as endothelial cells, epidermal cells, and fibroblasts) upon stimulation. In some embodiments, cytokines modulate the immune response by regulating cell growth, differentiation, and effects through binding to corresponding receptors. In some embodiments, cytokines include low molecular weight soluble proteins produced by cells upon induction by immunogen, mitogen, or other stimulants. In some embodiments, cytokines have functions in modulating innate and adaptive immunity, hematopoiesis, cell growth, APSC pluripotent cells, damaged tissue repair, etc. In some embodiments, cytokines encompass interleukins, interferons, tumor necrosis factor superfamily, colony-stimulating factors, chemokines, growth factors, and the like. In some specific embodiments, the "cytokines" in the present invention include those required for *in vitro* culture of T cells or for maintaining an optimal state. As used herein, "immune cell-activating cytokine" refers to cytokine that plays a positive role in responses of proliferation, differentiation, growth, survival, and maintenance of effector cells (such as T cells or B cells) of immune cells.

As used herein, "culture medium" refers to any liquid, solid, or semi-solid that can provide essential nutrients (including inorganic and organic substances) for *in vitro* survival of cells. In some embodiments, the culture medium can provide culture conditions that enable cell survival under serum-free conditions. In some embodiments, the serum-free culture medium is OpTmizer^{™} CTSTM (LifeTech), Immunocult^{™} XF (Stemcell technologies), CellGro^{™} (CellGenix), TexMacsTM (Miltenyi), StemlineTM (Sigma), Xvivo15TM (Lonza), PrimeXV (Irvine Scientific), StemXVivo or (RandD). The serum-free culture medium can be supplemented with serum replacements, such as ICSR (immune cell serum replacement) from LifeTech. The proportion of the serum replacement (e.g., ICSR) added can be, for example, up to 5%, such as about 1%, 2%, 3%, 4%, or 5%. In some embodiments, the serum-free culture medium can be supplemented with serum, such as human serum, for example, human AB serum. In some embodiments, the serum is human serum that allows natural clotting after collection, e.g., non-clotted (OTC) serum. In some embodiments, the serum is plasma-derived human serum. The plasma-derived serum can be produced by defibrating pooled human plasma collected in the presence of an anticoagulant (such as sodium citrate). In some embodiments, the culture medium is a culture medium suitable for T cell culture (e.g., Minimal Essential Media, α-MEM, RPMI 1640 culture medium, AIM-V, DMEM, F-12, or X-vivo 15 (Lonza), X-Vivo 20, OpTimizer, and IMDM), and the culture medium can contain factors essential for cell proliferation and viability maintenance. In some embodiments, the culture medium includes serum (e.g., fetal bovine or human serum), insulin, IFNγ, IL-7, IL-12, IL-15, and TNFα, or any other additives known to those skilled in the art for cell growth. Furthermore, unless otherwise specified, the culture medium and serum in the present invention explicitly excludes interleukin-2 (IL-2). Other additives for cell growth include, but are not limited to, surfactants, human plasma protein products, and reducing agents (such as N-acetyl-cysteine and 2-mercaptoethanol). The culture medium may include, but is not limited to, RPMI 1640, AIM-V, DMEM, MEM, α-MEM, F-12, X-Vivo 15, XVivo20, OpTimizer, and IMDM, added with amino acids, sodium pyruvate, and vitamins, serum-free or supplemented with an appropriate amount of serum (or plasma) or a set of defined hormones, and/or an amount of cytokines sufficient for T cell growth and expansion. In some embodiments, the "culture medium" of the present invention can further include antibiotics (e.g., penicillin and streptomycin) that are only contained in the experimental culture but not in the cell culture to be injected into a subject.

As used herein, the term "cell population" is a composition comprising one or more types of cells. In some embodiments, each cell in the "cell population" has one or more common characteristics, for example, having the same karyotype, having a similar function, falling within a certain same volume range, and having one or more identical cell markers (i.e., positive for the one or more identical cell markers), or lacking one or more specific cell markers (i.e., negative for the one or more specific cell markers). Herein, "lacking" or "negative" may not necessarily mean absolute deletion of the marker(s). Those skilled in the art could readily compare cells with positive and/or negative controls, and/or set a predetermined threshold, and when the cells are expressed at a level lower than the predetermined threshold or lower than the predetermined threshold level using conventional detection methods (e.g., using flow cytometry), the cells are classified as "lacking" or negative for the marker(s). For example, in some embodiments, the cell population is a T-cell population. In some embodiments, the cell population is PBMCs.

As used herein, the term "chimeric antigen receptor" or "CAR" refers to a set of engineered polypeptides or proteins that, when in immune effector cells, bind to specific antigens on target cells to generate intracellular signals, which activate downstream pathways of the cells where the receptors are located, thereby initiating a killing effect of the immune effector cells on the target cells. The immune effector cells include, but are not limited to, NK cells, macrophages, neutrophils, T cells, etc. The CAR typically includes at least one extracellular antigen-binding domain, a transmembrane domain, and a cytoplasmic signaling domain. The extracellular antigen-binding domain is capable of specifically recognizing an antigen, and non-limiting examples include a single-chain variable fragment (scFv) derived from an antibody, a fragment antigen-binding region (Fab) selected from a library, a single-domain fragment or a natural ligand binding to a cognate receptor thereof, and a target-specific recognition domain artificially designed for recognizing a specific target, such as fibronectin type III (FN3) domain combinations, and target-specific designed ankyrin repeat proteins (DARPins) recognizing specific targets. In some embodiments, the extracellular antigen-binding region can include a scFv, a Fab, or a natural ligand, as well as any derivatives thereof. An extracellular antigen-binding region may refer to a molecule other than an intact antibody, which can include a portion of an intact antibody and can bind to an antigen to which the intact antibody binds. Examples of antibody fragments can include, but are not limited to, Fv, Fab, Fab', Fab'-SH, F(ab')2; diabody, linear antibody; a single-chain antibody molecule (e.g. scFv), wherein the scFv can be a scFv of a murine antibody, a whole human antibody or a chimeric human-murine antibody, and can also be a single domain antibody such as a shark, a lama or a camel antibody; and polyclonal antibody formed from antibody fragments. The "signaling domain" typically includes immune-receptor tyrosine-based activation motifs (ITAM), with a basic composition of YXXL/V, wherein Y is tyrosine, L/V refers to leucine or valine, and X can be any amino acid. Upon binding of a receptor to a corresponding ligand, tyrosine within ITAM associated therewith can be phosphorylated by membrane-associated protein tyrosine kinases PTKs, thereby recruiting other free intracellular protein kinases or adaptor proteins, and propagating activation signals into cells. In some embodiments, the intracellular signaling domain of TCRζ (CD3ζ) or FcεRIγ is selected as the "signaling domain". As used herein, the "co-stimulatory domain," also referred to as "co-stimulatory signaling domain," is used primarily to provide co-stimulatory signals for enhancing the ability of immune cells, including, for example, for enhancing proliferation, survival, and/or development of memory cells. In some embodiments, the "co-stimulatory domain" is selected from intracellular domains of CD28, 4-1BB (CD137), OX40 (CD134), etc. As used herein, the "transmembrane domain," also known as "transmembrane region," refers to a protein structure region which has thermodynamical stability and is anchored within the cell membrane. The transmembrane domain can be obtained from a native protein, for example, from the transmembrane domain of the T cell receptor (TCR). In some embodiments, the transmembrane domain is selected from the transmembrane domains of CD4, CD8α, CD28, and CD3ζ.

In some embodiments, the extracellular antigen-binding region of the "chimeric antigen receptor" or "CAR" structure can recognize molecules of interest expressed on a surface of solid tumors, blood system tumor cells/tissues, wherein the molecules of interest include, but are not limited to, CD19, CD20, CD22, CD33, CLL-1 (CLEC12A), CD7, CD5, CD70, CD123, CEACAM5, CEACAM6, CEACAM7, Mesothelin, MUC1, CLDN18.2, CDH17, Trop2, BCMA, NKG2D, PDL1, EGFR, EGFRVIII, PSCA, PSMA, MUC16, CD133, GD2, IL13R2, B7H3, Her2, CD30, SLAMF7, CD38, GPC3, WT1, AFP, FOLR1, c-Met, LeY (Lewis Y antibody), L1CAM (L1 cell adhesion molecule), MAGE (melanoma antigen), MAGE-A1 (melanoma-associated antigen 1) or TAG-72, etc.

In some embodiments, the structure of the "chimeric antigen receptor" or "CAR" includes an antigen recognition domain (such as ScFv), a hinge structure (e.g., 8hinge derived from human CD8), a transmembrane structure (e.g., CD8TM derived from human CD8 transmembrane region), and an intracellular signaling domain, wherein the intracellular signaling domain may be a primary intracellular signaling domain comprising only the immunoreceptor tyrosine-based activation motif or ITAM, wherein examples of ITAM-containing primary cytoplasmic signaling sequences include but are not limited to CD3ζ, FcRy, FcRβ, CD3γ, CD3δ, CD3ε, CD5, CD22, CD79a, CD79b, ICOS (CD278), FcεRI, CD66d, DAP10, DAP12, etc., one or two or more co-stimulatory signaling domains may also be included in addition to the primary intracellular signaling domains, and the co-stimulatory signaling domain can be any one or more molecules and functional variants derived therefrom selected from the group consisting of: CD28, 41BB, OX40, CD27, DAP10, 2B4 (SLAMF4, CD244), CD3y, CD3δ, FcεRI, CD2, CD16, TCRζ, FcRβ, CD30, CD40, ICOS, LFA-1, IL-2 receptor, Fcγ receptor, KIRDS2, SLAMF7, NKp80 (KLRF1), signaling lymphocyte activation molecule (SLAM protein), KIRDS2, SLAMF7, NKp80 (KLRF1), NKp44, NKp30, NKp46, DAP12, NKG2D, NKG2C, TNFR2, TRANCE/RANKL, LFA-1(CD11a/CD18), GITR, BAFFR, LIGHT, HVEM(LIGHTR), and the like. The functional derivative molecule is an amino acid or nucleic acid sequence that has at least about 85%, 90%, 95%, or 99% sequence identity to the above molecule.

The "chimeric antigen receptor" or "CAR" may encompass a variety of structures, for example, containing secretable or membrane-expressed cytokines or antibody gene sequences; structures capable of regulated activation or inactivation, structures for regulating activation or inactivation including: a suicide switch, such as inducible caspase-9 (iCasp9), thymidine kinase (HSV-TK) and suicide epitope in herpes simplex virus, truncated EGFR (EGFRt), and Fas-FasL apoptosis domain; inducible CAR structures: peptide neo-epitopes (PNE), fluorescein (FITC), 10 amino acids (5B9-tag), FITC-HM-3 bifunctional molecules (FHBM) and scFv fused to antibody, streptavidin-2 (mSA2) biotin-binding domains, leucine ZipFv, VIPER CAR induction structure, biotin-bindingimmune receptor (BBIR) systems; and "logic gate" regulatory systems incorporating SynNotch receptors, etc.

As used herein, the term "C-CAR" refers to a preparation process in which an entire preparation cycle of CAR-T cells is greater than or equal to 7 days from start of T-cell activation, as well as a CAR-T product prepared using this preparation process.

As used herein, the term "P-CAR" refers to the rapid preparation process in the present invention and a CAR-T product prepared using this process.

In some embodiments, the structure of the "chimeric antigen receptor" or "CAR" may further comprise a chimeric fusion protein containing an extracellular antigen-recognition domain, a transmembrane domain, and an intracellular signaling domain. The extracellular antigen recognition domain of the chimeric fusion protein can be a complete and continuous extracellular segment of an expressed molecule/polypeptide, or a fusion form of the expressed molecule with other polypeptides, such as those derived from human CD8 or CD4.

The artificial/engineered TCR receptor herein refers to a specific receptor on a surface of a native or engineered T cell, which mainly relies on affinity-optimized or pure native TCR to recognize an antigen presented by a tumor MHC molecule, and transmits a stimulating signal to the inside of a cell through a TCR-CD3 complex.

### Method of Preparing Engineered Immune Cells

In one aspect, the present invention relates to a method of preparing engineered immune cells, including:
step (i): contacting a cell population with a CD3/TCR complex agonist;
step (ii): introducing a nucleic acid molecule into the cell population via transduction to yield cells comprising the nucleic acid molecule; and
step (iii): culturing the cell population for a period of time, and harvesting the cell population, the cell population at this time being the engineered immune cells.

As used herein, the "engineered immune cells" refer to engineered immune cells, e.g., into which exogenous nucleic acids or proteins are introduced or inserted. Herein, the immune cell is a collective term for cells involved in the immune response of an organism, including, but not limited to, granulocytes, macrophages, monocytes, dendritic cells (DC), natural killer (NK) cells, T lymphocytes (or T cells), B lymphocytes (or B cells), plasma cells, and the like. In the present invention, unless otherwise specified, the term "immune cell" further includes precursor cells of the above immune cells, such as progenitor cells or stem cells thereof, for example, lymphoblasts, lymphoid stem cells, etc.

In some embodiments, the "cell population" in the present invention is a population of cells including immune cells and/or precursor immune cells. In some embodiments, the cell population is derived from human peripheral blood. In some embodiments, the cell population comprises T cells. In some embodiments, the T cells are derived from PBMCs. In some embodiments, the cell population is peripheral blood mononuclear cells (PBMCs). In some embodiments, the cell population is a T-cell population, i.e., a cell population composed of a single or multiple subtypes of T cells.

In some embodiments, the nucleic acid molecule encodes the chimeric antigen receptor (CAR). In some embodiments, the nucleic acid molecule encodes the T cell receptor (TCR). In some embodiments, the cell population comprises T cells, the nucleic acid molecule encodes the CAR, and the engineered immune cells are CAR-T cells. In some embodiments, the cell population is PBMCs, the nucleic acid molecule encodes the CAR, and the engineered immune cells are CAR-T cells. In some embodiments, the cell population comprises T cells, the nucleic acid molecule encodes the TCR, and the engineered immune cells are TCR-T cells. In some embodiments, the cell population is PBMCs, the nucleic acid molecule encodes the TCR, and the engineered immune cells are CAR-T cells.

In some embodiments, the cell population is in contact with an immune cell-activating cytokine in step i), step ii), and/or step iii), or throughout an entire preparation process, wherein the immune cell-activating cytokine excludes IL-2. The method of the present invention can achieve of the effect of eliminating the use IL-2 in the process of preparing the engineered immune cells. However, those skilled in the art would know that on the basis of preparing the engineered immune cell in the present invention, the engineered immune cells can still be prepared when IL-2 is added in a certain stage of the preparation process, which still falls within the scope of technical solutions of the present invention. In some embodiments, the immune cell-activating cytokine comprises one or more selected from the group consisting of IL-7, IL-15, IL-21, IL-12, IL-18, and IL-6. In some embodiments, the immune cell-activating cytokine comprises a combination of IL7 and IL21. In some embodiments, the immune cell-activating cytokine is a combination of IL-7 and IL-21 and excludes other immune activating cytokines. In some embodiments, when the cell population is in contact with the immune cell-activating cytokine, a concentration of IL-7 is 2.5-10 ng/ml, such as 3 ng/ml, 3.5 ng/ml, 4 ng/ml, 4.5 ng/ml, 5 ng/ml, 5.1 ng/ml, 5.2 ng/ml, 5.3 ng/ml, 5.4 ng/ml, 5.5 ng/ml, 5.6 ng/ml, 5.7 ng/ml, 5.8 ng/ml, 5.9 ng/ml, 6 ng/ml, 6.5 ng/ml, 7 ng/ml, 7.5 ng/ml, 8 ng/ml, 8.5 ng/ml, 9 ng/ml, 9.5 ng/ml, or any value in the range of 2.5-10 ng/ml. In some embodiments, an initial concentration of IL-7 when in contact with the cell population is 2.5-10 ng/ml, such as 3 ng/ml, 3.5 ng/ml, 4 ng/ml, 4.5 ng/ml, 5 ng/ml, 5.1 ng/ml, 5.2 ng/ml, 5.3 ng/ml, 5.4 ng/ml, 5.5 ng/ml, 5.6 ng/ml, 5.7 ng/ml, 5.8 ng/ml, 5.9 ng/ml, 6 ng/ml, 6.5 ng/ml, 7 ng/ml, 7.5 ng/ml, 8 ng/ml, 8.5 ng/ml, 9 ng/ml, 9.5 ng/ml, or any value in the range of 2.5-10 ng/ml, and the concentration may change gradually over the time of contact, for instance, decreasing. In some embodiments, when the cell population is in contact with the immune cell-activating cytokine, a concentration of IL21 is 12.5-50 ng/ml, such as 13 ng/ml, 13.5 ng/ml, 14 ng/ml, 14.5 ng/ml, 15 ng/ml, 15.5 ng/ml, 16 ng/ml, 16.5 ng/ml, 17 ng/ml, 17.5 ng/ml, 18 ng/ml, 18.5 ng/ml, 19 ng/ml, 19.5 ng/ml, 20 ng/ml, 20.5 ng/ml, 21 ng/ml, 21.5 ng/ml, 22 ng/ml, 22.5 ng/ml, 23 ng/ml, 23.5 ng/ml, 24 ng/ml, 24.5 ng/ml, 25 ng/ml, 25.5 ng/ml, 26 ng/ml, 26.5 ng/ml, 27 ng/ml, 27.5 ng/ml, 28 ng/ml, 28.5 ng/ml, 29 ng/ml, 29.5 ng/ml, 30 ng/ml, 30.5 ng/ml, 31 ng/ml, 31.5 ng/ml, 32 ng/ml, 32.5 ng/ml, 33 ng/ml, 33.5 ng/ml, 34 ng/ml, 34.5 ng/ml, 35 ng/ml, 35.5 ng/ml, 36 ng/ml, 36.5 ng/ml, 37 ng/ml, 37.5 ng/ml, 38 ng/ml, 38.5 ng/ml, 39 ng/ml, 39.5 ng/ml, 40 ng/ml, 40.5 ng/ml, 41 ng/ml, 41.5 ng/ml, 42 ng/ml, 42.5 ng/ml, 43 ng/ml, 43.5 ng/ml, 44 ng/ml, 44.5 ng/ml, 45 ng/ml, 45.5 ng/ml, 46 ng/ml, 46.5 ng/ml, 47 ng/ml, 47.5 ng/ml, 48 ng/ml, 48.5 ng/ml, 49 ng/ml, 49.5 ng/ml, or any value in the range of 12.5-50 ng/ml. In some embodiments, an initial concentration of IL-21 when in contact with the cell population is 12.5-50 ng/ml, such as 13 ng/ml, 13.5 ng/ml, 14 ng/ml, 14.5 ng/ml, 15 ng/ml, 15.5 ng/ml, 16 ng/ml, 16.5 ng/ml, 17 ng/ml, 17.5 ng/ml, 18 ng/ml, 18.5 ng/ml, 19 ng/ml, 19.5 ng/ml, 20 ng/ml, 20.5 ng/ml, 21 ng/ml, 21.5 ng/ml, 22 ng/ml, 22.5 ng/ml, 23 ng/ml, 23.5 ng/ml, 24 ng/ml, 24.5 ng/ml, 25 ng/ml, 25.5 ng/ml, 26 ng/ml, 26.5 ng/ml, 27 ng/ml, 27.5 ng/ml, 28 ng/ml, 28.5 ng/ml, 29 ng/ml, 29.5 ng/ml, 30 ng/ml, 30.5 ng/ml, 31 ng/ml, 31.5 ng/ml, 32 ng/ml, 32.5 ng/ml, 33 ng/ml, 33.5 ng/ml, 34 ng/ml, 34.5 ng/ml, 35 ng/ml, 35.5 ng/ml, 36 ng/ml, 36.5 ng/ml, 37 ng/ml, 37.5 ng/ml, 38 ng/ml, 38.5 ng/ml, 39 ng/ml, 39.5 ng/ml, 40 ng/ml, 40.5 ng/ml, 41 ng/ml, 41.5 ng/ml, 42 ng/ml, 42.5 ng/ml, 43 ng/ml, 43.5 ng/ml, 44 ng/ml, 44.5 ng/ml, 45 ng/ml, 45.5 ng/ml, 46 ng/ml, 46.5 ng/ml, 47 ng/ml, 47.5 ng/ml, 48 ng/ml, 48.5 ng/ml, 49 ng/ml, 49.5 ng/ml, or any value in the range of 12.5-50 ng/ml, and the concentration may change gradually over the time of contact, for instance, decreasing.

In some embodiments, the cell population is in contact with the immune cell-activating cytokine in step i), step ii), and/or step iii), or throughout an entire preparation process, wherein the immune cell-activating cytokine comprises IL-2. In some embodiments, a concentration of the IL-2 is 100 IU/ml-500 IU/ml, 150 IU/ml-450 IU/ml, 200 IU/ml-400 IU/ml, or 250 IU/ml-350 IU/ml.

In some embodiments, the cell population is not in contact with a CD28 agonist in step i), steps i) and ii), steps i), ii), and iii), or throughout an entire preparation process. In some embodiments, the cell population is not in contact with an agonist for cell surface co-stimulatory molecules in step i), steps i) and ii), steps i), ii), and iii), or throughout an entire preparation process, wherein the co-stimulatory molecules include: CD28, ICOS, CD27, HVEM, LIGHT, CD40, 4-1BB, OX40, DR3, GITR, CD30, TIM1, CD2, and CD226.

As used herein, the term "preparation process" refers to a process of preparing the engineered immune cells using the method of preparing engineered immune cells, including all steps of the method of preparing the engineered immune cells, and other necessary steps of preparing the engineered immune cells. Steps of preparing engineered immune cells, such as CAR-T or TCR-T, are known in the art, such as those described in CN106459917B, WO2003095663A3, and US7446190B2. In some embodiments, an "entire preparation process" refers to an entire process from the moment the cell population is in contact with the CD3/TCR complex agonist to step iii) of harvesting the cell population. In some embodiments, the "entire preparation process" refers to an entire process from separation of the cell population from an organism (for example, mammal, such as human) to step iii) of harvesting the cell population.

In some embodiments, the CD3/TCR complex agonist comprises an anti-CD3 antibody. Herein, the anti-CD3 antibody can be coated on a bead, such as a magnetic bead, or dispersed in a liquid. In some embodiments, a concentration of the anti-CD3 antibody in contact with the cell population in step i) is ≥ 25 ng/ml, for example, 25-200 ng/ml or any value therein, such as 26 ng/ml, 27 ng/ml, 28 ng/ml, 29 ng/ml, 30 ng/ml, 31 ng/ml, 32 ng/ml, 33 ng/ml, 34 ng/ml, 35 ng/ml, 36 ng/ml, 37 ng/ml, 38 ng/ml, 39 ng/ml, 40 ng/ml, 41 ng/ml, 42 ng/ml, 43 ng/ml, 44 ng/ml, 45 ng/ml, 46 ng/ml, 47 ng/ml, 48 ng/ml, 49 ng/ml, 50 ng/ml, 51 ng/ml, 52 ng/ml, 53 ng/ml, 54 ng/ml, 55 ng/ml, 56 ng/ml, 57 ng/ml, 58 ng/ml, 59 ng/ml, 60 ng/ml, 61 ng/ml, 62 ng/ml, 63 ng/ml, 64 ng/ml, 65 ng/ml, 66 ng/ml, 67 ng/ml, 68 ng/ml, 69 ng/ml, 70 ng/ml, 71 ng/ml, 72 ng/ml, 73 ng/ml, 74 ng/ml, 75 ng/ml, 76 ng/ml, 77 ng/ml, 78 ng/ml, 79 ng/ml, 80 ng/ml, 81 ng/ml, 82 ng/ml, 83 ng/ml, 84 ng/ml, 85 ng/ml, 86 ng/ml, 87 ng/ml, 88 ng/ml, 89 ng/ml, 90 ng/ml, 91 ng/ml, 92 ng/ml, 93 ng/ml, 94 ng/ml, 95 ng/ml, 96 ng/ml, 97 ng/ml, 98 ng/ml, 99 ng/ml, 100 ng/ml, 101 ng/ml, 102 ng/ml, 103 ng/ml, 104 ng/ml, 105 ng/ml, 106 ng/ml, 107 ng/ml, 108 ng/ml, 109 ng/ml, 110 ng/ml, 111 ng/ml, 112 ng/ml, 113 ng/ml, 114 ng/ml, 115 ng/ml, 116 ng/ml, 117 ng/ml, 118 ng/ml, 119 ng/ml, 120 ng/ml, 121 ng/ml, 122 ng/ml, 123 ng/ml, 124 ng/ml, 125 ng/ml, 126 ng/ml, 127 ng/ml, 128 ng/ml, 129 ng/ml, 130 ng/ml, 131 ng/ml, 132 ng/ml, 133 ng/ml, 134 ng/ml, 135 ng/ml, 136 ng/ml, 137 ng/ml, 138 ng/ml, 139 ng/ml, 140 ng/ml, 141 ng/ml, 142 ng/ml, 143 ng/ml, 144 ng/ml, 145 ng/ml, 146 ng/ml, 147 ng/ml, 148 ng/ml, 149 ng/ml, 150 ng/ml, 151 ng/ml, 152 ng/ml, 153 ng/ml, 154 ng/ml, 155 ng/ml, 156 ng/ml, 157 ng/ml, 158 ng/ml, 159 ng/ml, 160 ng/ml, 161 ng/ml, 162 ng/ml, 163 ng/ml, 164 ng/ml, 165 ng/ml, 166 ng/ml, 167 ng/ml, 168 ng/ml, 169 ng/ml, 170 ng/ml, 171 ng/ml, 172 ng/ml, 173 ng/ml, 174 ng/ml, 175 ng/ml, 176 ng/ml, 177 ng/ml, 178 ng/ml, 179 ng/ml, 180 ng/ml, 181 ng/ml, 182 ng/ml, 183 ng/ml, 184 ng/ml, 185 ng/ml, 186 ng/ml, 187 ng/ml, 188 ng/ml, 189 ng/ml, 190 ng/ml, 191 ng/ml, 192 ng/ml, 193 ng/ml, 194 ng/ml, 195 ng/ml, 196 ng/ml, 197 ng/ml, 198 ng/ml, or 199 ng/ml. In some embodiments, an initial concentration of the CD3 in contact with the cell population is ≥ 25 ng/ml, for example, 25-200 ng/ml or any value therein, such as 26 ng/ml, 27 ng/ml, 28 ng/ml, 29 ng/ml, 30 ng/ml, 31 ng/ml, 32 ng/ml, 33 ng/ml, 34 ng/ml, 35 ng/ml, 36 ng/ml, 37 ng/ml, 38 ng/ml, 39 ng/ml, 40 ng/ml, 41 ng/ml, 42 ng/ml, 43 ng/ml, 44 ng/ml, 45 ng/ml, 46 ng/ml, 47 ng/ml, 48 ng/ml, 49 ng/ml, 50 ng/ml, 51 ng/ml, 52 ng/ml, 53 ng/ml, 54 ng/ml, 55 ng/ml, 56 ng/ml, 57 ng/ml, 58 ng/ml, 59 ng/ml, 60 ng/ml, 61 ng/ml, 62 ng/ml, 63 ng/ml, 64 ng/ml, 65 ng/ml, 66 ng/ml, 67 ng/ml, 68 ng/ml, 69 ng/ml, 70 ng/ml, 71 ng/ml, 72 ng/ml, 73 ng/ml, 74 ng/ml, 75 ng/ml, 76 ng/ml, 77 ng/ml, 78 ng/ml, 79 ng/ml, 80 ng/ml, 81 ng/ml, 82 ng/ml, 83 ng/ml, 84 ng/ml, 85 ng/ml, 86 ng/ml, 87 ng/ml, 88 ng/ml, 89 ng/ml, 90 ng/ml, 91 ng/ml, 92 ng/ml, 93 ng/ml, 94 ng/ml, 95 ng/ml, 96 ng/ml, 97 ng/ml, 98 ng/ml, 99 ng/ml, 100 ng/ml, 101 ng/ml, 102 ng/ml, 103 ng/ml, 104 ng/ml, 105 ng/ml, 106 ng/ml, 107 ng/ml, 108 ng/ml, 109 ng/ml, 110 ng/ml, 111 ng/ml, 112 ng/ml, 113 ng/ml, 114 ng/ml, 115 ng/ml, 116 ng/ml, 117 ng/ml, 118 ng/ml, 119 ng/ml, 120 ng/ml, 121 ng/ml, 122 ng/ml, 123 ng/ml, 124 ng/ml, 125 ng/ml, 126 ng/ml, 127 ng/ml, 128 ng/ml, 129 ng/ml, 130 ng/ml, 131 ng/ml, 132 ng/ml, 133 ng/ml, 134 ng/ml, 135 ng/ml, 136 ng/ml, 137 ng/ml, 138 ng/ml, 139 ng/ml, 140 ng/ml, 141 ng/ml, 142 ng/ml, 143 ng/ml, 144 ng/ml, 145 ng/ml, 146 ng/ml, 147 ng/ml, 148 ng/ml, 149 ng/ml, 150 ng/ml, 151 ng/ml, 152 ng/ml, 153 ng/ml, 154 ng/ml, 155 ng/ml, 156 ng/ml, 157 ng/ml, 158 ng/ml, 159 ng/ml, 160 ng/ml, 161 ng/ml, 162 ng/ml, 163 ng/ml, 164 ng/ml, 165 ng/ml, 166 ng/ml, 167 ng/ml, 168 ng/ml, 169 ng/ml, 170 ng/ml, 171 ng/ml, 172 ng/ml, 173 ng/ml, 174 ng/ml, 175 ng/ml, 176 ng/ml, 177 ng/ml, 178 ng/ml, 179 ng/ml, 180 ng/ml, 181 ng/ml, 182 ng/ml, 183 ng/ml, 184 ng/ml, 185 ng/ml, 186 ng/ml, 187 ng/ml, 188 ng/ml, 189 ng/ml, 190 ng/ml, 191 ng/ml, 192 ng/ml, 193 ng/ml, 194 ng/ml, 195 ng/ml, 196 ng/ml, 197 ng/ml, 198 ng/ml, or 199 ng/ml, and the concentration may change gradually over the time of contact, for instance, decreasing.

As described in the above, any expression "initial concentration when in contact with the cell population" or "concentration when in contact with the cell population" for the protein refers to an environment where the cell population is located, for example, a concentration of the protein in a culture medium where the cell population is located, such as protein concentration of anti-CD3 antibody, IL21, or IL7. Herein, the "concentration of the protein in a culture medium" refers to a ratio of a mass of the protein contained in the culture medium to a volume of the culture medium. Due to reasons such as culture medium evaporation or protein degradation, the concentration may vary with the culture time, which still belongs to the scope of the present invention, provided that it is still within the above concentration range, or it can be maintained at the above concentration within a period of time.

Using the method of preparing engineered immune cells of the present invention can allow completion of the preparation of the engineered immune cells within a short period of time. For example, in some embodiments, the duration from start of step i) to harvest of the cell population only requires 1 to 4 days. In some embodiments, the duration from start of step i) to harvest of the cell population only requires 1 to 2 days. That is, the method of preparing engineered immune cells of the present invention can allow completion of the preparation of the engineered immune cells within 4 days, and even 1-2 days. This time range should not be construed as limitation to the present invention, because those skilled in the art would be aware that if the cell population is further cultured, for example, changing durations of steps i), ii), and/or iii), to make the duration from start of step i) to harvest of the cell population exceed 4 days, the engineered immune cells can still be obtained. Thus, an optional duration from the start of step i) to the harvest of the cell population is still encompassed within the scope of the present invention. The durations provided for steps i), ii), and iii) and the duration from the start of step i) to the harvest of the cell population in the present invention are merely optional or preferred solutions with consideration to temporal and economic efficiency or the quality of the cell population. In some embodiments, steps i), ii), and/or iii) may be partially or fully overlapping, or may be fully non-overlapping. For example, in some embodiments, the CD3/TCR complex agonist in step i) can be present throughout the entire preparation process from step i), and the contact of the CD3/TCR complex agonist with the cell population can be terminated at any time during the entire preparation process (for example, before, at, or following start of step ii)). In some embodiments, step ii) may start prior to, subsequent to or concurrently with with step i). In some embodiments, step ii) precedes step i). That is, irrespective of any temporal overlap between steps (i) and (ii) or a sequential order of steps (i) and (ii), they are all encompassed within the scope of the present invention.

In some embodiments, step i) lasts for 12 to 24 hours, for example, 13, 14, 15, 16, 17, 18, 19, 20, 21, 22, or 23 hours. In some embodiments, the step i) lasts for greater than 12 to 24 hours, for example, 13.5, 14.5, 15.5, 16.5, 17.5, 18.5, 19.5, 20.5, 21.5, 22.5, or 23.5 hours.

In some embodiments, the duration from the start of step ii) to the harvest of the cell population in the method is ≤ 72 hours or ≤ 48 hours. In some embodiments, the duration from the start of step ii) to the harvest of the cell population in the method is ≥6 hours. In some embodiments, the duration from the start of step ii) to the harvest of the cell population in the method is ≥ 6 hours and ≤ 72 hours, such as 7 hours, 8 hours, 9 hours, 10 hours, 11 hours, 12 hours, 13 hours, 14 hours, 15 hours, 16 hours, 17 hours, 18 hours, 19 hours, 20 hours, 21 hours, 22 hours, 23 hours, 24 hours, 25 hours, 26 hours, 27 hours, 28 hours, 29 hours, 30 hours, 31 hours, 32 hours, 33 hours, 34 hours, 35 hours, 36 hours, 37 hours, 38 hours, 39 hours, 40 hours, 41 hours, 42 hours, 43 hours, 44 hours, 45 hours, 46 hours, 47 hours, 48 hours, 49 hours, 50 hours, 51 hours, 52 hours, 53 hours, 54 hours, 55 hours, 56 hours, 57 hours, 58 hours, 59 hours, 60 hours, 61 hours, 62 hours, 63 hours, 64 hours, 65 hours, 66 hours, 67 hours, 68 hours, 69 hours, 70 hours, or 71 hours, that is, harvesting the cell population at any time point from 6 hours to 72 hours after the start of step ii) is encompassed within the scope of the present invention.

In some embodiments, a duration from step i) until start of step ii) is 12 to 24 hours, and a duration from the start of step ii) to the harvest of the cell population is 14-28 hours; that is, step i) starts prior to step ii) and continues until start of step ii) or a certain time point after the start of step ii), the duration from the start of step i) to the start of step ii) is 12 to 24 hours, such as 13, 14, 15, 16, 17, 18, 19, 20, 21, 22, or 23 hours, and the duration from the start of step ii) to the harvest of the cell population is 14-28 hours, such as 15 hours, 16 hours, 17 hours, 18 hours, 19 hours, 20 hours, 21 hours, 22 hours, 23 hours, 24 hours, 25 hours, 26 hours, or 27 hours. In some embodiments, step i) lasts for 12 to 24 hours until the start of step ii), the duration from the start of step ii) to the harvest of the cell population is 14-28 hours, and the duration from the start of step i) to the harvest of the cell population is 1 to 4 days, such as 2 days and 3 days.

### Composition

In the second aspect, the present invention provides a composition for preparing engineered immune cells. In some embodiments, the engineered immune cells are T cells or precursor cells thereof. In some embodiments, the engineered immune cells are CAR-T cells or TCR-T cells.

The composition comprises a CD3/TCR complex agonist and an immune cell-activating cytokine, wherein the immune cell-activating cytokine excludes or comprises IL-2. In some embodiments, the CD3/TCR complex agonist comprises an anti-CD3 antibody. In some embodiments, a concentration of the anti-CD3 antibody is ≥ 5 ng/ml, for example, 25-200 ng/ml or any value therein, such as 26 ng/ml, 27 ng/ml, 28 ng/ml, 29 ng/ml, 30 ng/ml, 31 ng/ml, 32 ng/ml, 33 ng/ml, 34 ng/ml, 35 ng/ml, 36 ng/ml, 37 ng/ml, 38 ng/ml, 39 ng/ml, 40 ng/ml, 41 ng/ml, 42 ng/ml, 43 ng/ml, 44 ng/ml, 45 ng/ml, 46 ng/ml, 47 ng/ml, 48 ng/ml, 49 ng/ml, 50 ng/ml, 51 ng/ml, 52 ng/ml, 53 ng/ml, 54 ng/ml, 55 ng/ml, 56 ng/ml, 57 ng/ml, 58 ng/ml, 59 ng/ml, 60 ng/ml, 61 ng/ml, 62 ng/ml, 63 ng/ml, 64 ng/ml, 65 ng/ml, 66 ng/ml, 67 ng/ml, 68 ng/ml, 69 ng/ml, 70 ng/ml, 71 ng/ml, 72 ng/ml, 73 ng/ml, 74 ng/ml, 75 ng/ml, 76 ng/ml, 77 ng/ml, 78 ng/ml, 79 ng/ml, 80 ng/ml, 81 ng/ml, 82 ng/ml, 83 ng/ml, 84 ng/ml, 85 ng/ml, 86 ng/ml, 87 ng/ml, 88 ng/ml, 89 ng/ml, 90 ng/ml, 91 ng/ml, 92 ng/ml, 93 ng/ml, 94 ng/ml, 95 ng/ml, 96 ng/ml, 97 ng/ml, 98 ng/ml, 99 ng/ml, 100 ng/ml, 101 ng/ml, 102 ng/ml, 103 ng/ml, 104 ng/ml, 105 ng/ml, 106 ng/ml, 107 ng/ml, 108 ng/ml, 109 ng/ml, 110 ng/ml, 111 ng/ml, 112 ng/ml, 113 ng/ml, 114 ng/ml, 115 ng/ml, 116 ng/ml, 117 ng/ml, 118 ng/ml, 119 ng/ml, 120 ng/ml, 121 ng/ml, 122 ng/ml, 123 ng/ml, 124 ng/ml, 125 ng/ml, 126 ng/ml, 127 ng/ml, 128 ng/ml, 129 ng/ml, 130 ng/ml, 131 ng/ml, 132 ng/ml, 133 ng/ml, 134 ng/ml, 135 ng/ml, 136 ng/ml, 137 ng/ml, 138 ng/ml, 139 ng/ml, 140 ng/ml, 141 ng/ml, 142 ng/ml, 143 ng/ml, 144 ng/ml, 145 ng/ml, 146 ng/ml, 147 ng/ml, 148 ng/ml, 149 ng/ml, 150 ng/ml, 151 ng/ml, 152 ng/ml, 153 ng/ml, 154 ng/ml, 155 ng/ml, 156 ng/ml, 157 ng/ml, 158 ng/ml, 159 ng/ml, 160 ng/ml, 161 ng/ml, 162 ng/ml, 163 ng/ml, 164 ng/ml, 165 ng/ml, 166 ng/ml, 167 ng/ml, 168 ng/ml, 169 ng/ml, 170 ng/ml, 171 ng/ml, 172 ng/ml, 173 ng/ml, 174 ng/ml, 175 ng/ml, 176 ng/ml, 177 ng/ml, 178 ng/ml, 179 ng/ml, 180 ng/ml, 181 ng/ml, 182 ng/ml, 183 ng/ml, 184 ng/ml, 185 ng/ml, 186 ng/ml, 187 ng/ml, 188 ng/ml, 189 ng/ml, 190 ng/ml, 191 ng/ml, 192 ng/ml, 193 ng/ml, 194 ng/ml, 195 ng/ml, 196 ng/ml, 197 ng/ml, 198 ng/ml, or 199 ng/ml.

In some embodiments, the immune cell-activating cytokine comprises one or more selected from the group consisting of IL-7, IL-15, IL-21, IL-12, IL-18, and IL-6. In some embodiments, the immune cell-activating cytokine comprises a combination of IL-7 and IL-21. In some embodiments, the immune cell-activating cytokine comprises a combination of IL-7 and IL-21, excluding other immune cell-activating cytokines. In some embodiments, a concentration of IL-7 in the composition is 2.5-10 ng/ml, such as 3 ng/ml, 3.5 ng/ml, 4 ng/ml, 4.5 ng/ml, 5 ng/ml, 5.1 ng/ml, 5.2 ng/ml, 5.3 ng/ml, 5.4 ng/ml, 5.5 ng/ml, 5.6 ng/ml, 5.7 ng/ml, 5.8 ng/ml, 5.9 ng/ml, 6 ng/ml, 6.5 ng/ml, 7 ng/ml, 7.5 ng/ml, 8 ng/ml, 8.5 ng/ml, 9 ng/ml or 9.5 ng/ml, 2.5-10 ng/ml, or any value in the range of 2.5-10 ng/ml. In some embodiments, a concentration of IL21 in the composition is 12.5-50 ng/ml, such as 13 ng/ml, 13.5 ng/ml, 14 ng/ml, 14.5 ng/ml, 15 ng/ml, 15.5 ng/ml, 16 ng/ml, 16.5 ng/ml, 17 ng/ml, 17.5 ng/ml, 18 ng/ml, 18.5 ng/ml, 19 ng/ml, 19.5 ng/ml, 20 ng/ml, 20.5 ng/ml, 21 ng/ml, 21.5 ng/ml, 22 ng/ml, 22.5 ng/ml, 23 ng/ml, 23.5 ng/ml, 24 ng/ml, 24.5 ng/ml, 25 ng/ml, 25.5 ng/ml, 26 ng/ml, 26.5 ng/ml, 27 ng/ml, 27.5 ng/ml, 28 ng/ml, 28.5 ng/ml, 29 ng/ml, 29.5 ng/ml, 30 ng/ml, 30.5 ng/ml, 31 ng/ml, 31.5 ng/ml, 32 ng/ml, 32.5 ng/ml, 33 ng/ml, 33.5 ng/ml, 34 ng/ml, 34.5 ng/ml, 35 ng/ml, 35.5 ng/ml, 36 ng/ml, 36.5 ng/ml, 37 ng/ml, 37.5 ng/ml, 38 ng/ml, 38.5 ng/ml, 39 ng/ml, 39.5 ng/ml, 40 ng/ml, 40.5 ng/ml, 41 ng/ml, 41.5 ng/ml, 42 ng/ml, 42.5 ng/ml, 43 ng/ml, 43.5 ng/ml, 44 ng/ml, 44.5 ng/ml, 45 ng/ml, 45.5 ng/ml, 46 ng/ml, 46.5 ng/ml, 47 ng/ml, 47.5 ng/ml, 48 ng/ml, 48.5 ng/ml, 49 ng/ml, 49.5 ng/ml, or any value in the range of 12.5-50 ng/ml.

In some embodiments, the composition excludes a CD28 agonist. In some embodiments, the composition excludes any agonist for cell surface co-stimulatory molecules. In some embodiments, the cell surface co-stimulatory molecules include: CD28, ICOS, CD27, HVEM, LIGHT, CD40, 4-1BB, OX40, DR3, GITR, CD30, TIM1, CD2, and CD226.

In some embodiments, the composition is a culture medium, which, in addition to the CD3/TCR complex agonist and the immune cell-activating cytokine, further includes essential nutrients (including inorganic and organic substances) for *in vitro* survival of cells, such as water, amino acids, sodium pyruvate, and vitamins. In some embodiments, the essential nutrients further include nutrients (e.g., ICSRs), such as serum replacements, that enable cell survival under serum-free conditions. In some embodiments, the essential nutrients include serum, e.g., human serum or fetal bovine serum. In some embodiments, the composition is a commercial culture medium added with the CD3/TCR complex agonist and the immune cell-activating cytokine, wherein the commercial culture medium can be selected from the group consisting of, for example, OpTmizer^{™} CTS^{™}, Immunocult^{™} XF, CellGro^{™} (CellGenix), TexMacs^{™}, Stemline^{™}, Xvivo15^{™}, PrimeXV, StemXVivo, α-MEM, RPMI 1640, AIM-V, DMEM, F-12, X-vivo 15, X-Vivo 20, OpTmizer, and IMDM. In some embodiments, the culture medium further comprises one or more selected from the group consisting of insulin, IFNγ, IL-7, IL-12, IL-15, and TNFα, surfactants, human plasma protein preparations, and reducing agents (such as N-acetyl-cysteine and 2-mercaptoethanol), and the like.

In addition, the present invention further relates to a kit comprising the above composition, wherein the kit excludes IL-2 and CD28 agonist.

The present invention further relates to a use of the above composition and kit. In some embodiments, the use involves preparation of engineered immune cells. In some embodiments, the engineered immune cells comprise T cells. In some embodiments, the engineered immune cells are derived from PBMCs. In some embodiments, the engineered immune cells are CAR-T cells or TCR-T cells.

The present invention has beneficial effects as follows.
1) It is capable of completing the preparation of CAR-T cells within 48h, faster than conventional solutions.
2) The prepared CAR-T cells exhibit better *in vivo* efficacy and persistence.
3) The entire preparation process completely eliminates the need for the CD28 agonist, and the prepared CAR-T cells are safer than those from currently disclosed preparation solutions.
4) The solutions of the present invention further can be implemented without the addition of IL-2, further improving the safety of the CAR-T product.

It should be understood that the present invention encompasses various aspects, embodiments, and combinations of the aspects and/or embodiments described herein. The above description and the following examples are intended to illustrate, but not to limit, the scope of the present invention. Other aspects, improvements, and modifications within the scope of the present invention would be apparent to those skilled in the art to which the present invention pertains. Accordingly, those ordinarily skill in the art should recognize that the scope of the present invention also includes such improvements and modifications to the aspects and embodiments.

### Examples

Exemplary examples of the present invention are described below in conjunction with the drawings, in which various details of the examples of the present invention are included to facilitate understanding, and should be considered as merely exemplary. Therefore, those ordinarily skilled in the art should recognize that various changes and modifications could be made to the examples described herein without departing from the scope and spirit of the present invention. Likewise, descriptions of well-known features and structures are omitted from the following description for clarity and conciseness.

Relevant amino acid sequences of CAR structures used in the following examples are as shown in the Sequence Listing provided at the end.

### Example 1: Preparation Process for New CAR-T Cells

The conventional CAR-T cell preparation is divided into three procedures of cell activation, viral transduction, and cell harvest. A rapid preparation solution is divided into two stages of cell activation-viral transduction and viral transduction-cell harvest, wherein the cell activation-viral transduction refers to a duration spanning from addition of the culture reagent into cells until just before the viral transduction, and the viral transduction-cell harvest refers to a duration spanning from the addition of virus to the activated T cell culture for the viral transduction until the cell harvest. No removal of the activating agent is performed between the two procedures, and no removal of the virus is conducted from the viral transduction to the cell harvest. Currently, no existing technology can provide insights into whether culture time of the above two stages affects the ultimate functionality of CAR-T cells, necessitating our inventive exploration.

### 1.1 Confirmation of More Efficient Short-Duration CAR-T Preparation Solution

Mononuclear cells obtained through isolation or T cells sorted from mononuclear cells (which may also be the above cells having undergone recovery after cryopreservation) were activated using 50 ng/ml anti-CD3 antibody (Recombinant anti-Human CD3 mAb, GMP-A018), and cultured in the system containing 5 ng/ml IL-7 and 25 ng/ml IL-21 cytokines (SCGM serum-free culture medium, CellGenix, 20802-0500). After activation for varying durations (as in Table 1: Cell Activation-Viral Transduction), lentiviral transduction was performed. The cell harvest was completed at varying time after the viral transduction (as in Table 1: Viral Transduction-Cell Harvest). Four solutions of CAR-T preparation time, with varying durations for activation (Cell Activation-Viral Transduction) and viral transduction (Viral Transduction-Cell Harvest), were designed as listed in Table 1 to investigate an optimal rapid cell preparation solution.

**Table 1 CAR-T Cell Preparation Solutions (Time-based Optimization)**

| Group | Cell Activation-Viral Transduction | Viral Transduction-Cell Harvest | Total Preparation Time |
|---|---|---|---|
| Solution 1 | 0 h | 24 h | 1 day |
| Solution 2 | 6 h | 18 h | 1 day |
| Solution 3 | 12-24 h | 14-28 h | 2 days |
| Solution 4 | 24 h | 72 h | 4 days |

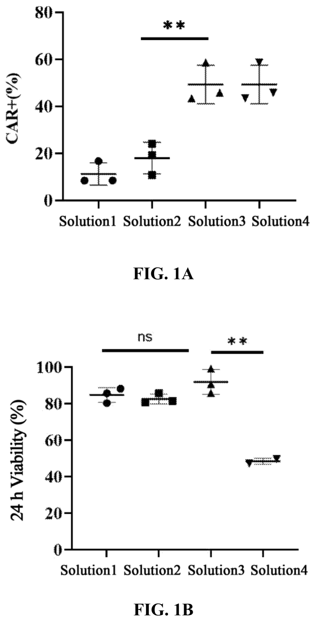

The CAR-T cells were prepared rapidly according to the four preparation solutions in the above, and the CAR positive rates and the cell viability after cryopreservation and recovery in the several solutions were compared. The CAR-T cells harvested from different preparation solutions were respectively resuspended in PBS solution containing 1% fetal bovine serum by volume. The collected cells were aliquoted, 100 µl per tube. 5 µl of PE-PL reagent was added to each tube of cells (Protein-L can recognize antibody light chain, and the light chain of the ScFv sequence of the CAR antigen recognition region could be recognized by Protein-L, thus allowing detection of the CAR positive rate using Protein-L), followed by incubation at 4 °C for 30 min, twice washing with PBS solution, and detection by a flow cytometer. Detection results of CAR expression positive rate are as shown in FIG. 1A, wherein in FIG. 1A the horizontal axis represents the CAR-T cells from different preparation solutions, and the vertical axis represents the CAR expression positive rates. The results demonstrate that solution 3 yielded an optimal CAR positive cell proportion compared with solutions 1 and 2.

Further, given that application scenarios of the CAR-T cells always involve cryopreservation, the prepared CAR-T cells require recovery prior to infusion into patients, and the CAR-T cell viability will affect calculation of real dose and *in vivo* efficacy, the antifreezing performance of the CAR-T cells prepared by different culture solutions is thus also critical for the functional performance of the CAR-T cells. The CAR-T cells prepared in the above were cryopreserved for 48 hours using the commercial cryostat (CryoStor ^{®}CS10). After the cryopreserved CAR-T cells were rapidly recovered in a water bath at 37 °C, a certain amount of the cells were subjected to the cell viability assay using AOPI, and the detection results are as shown in FIG. 1B, wherein the horizontal axis represents the CAR-T cells from different preparation solutions, and the vertical axis represents the proportions of viable CAR-T cells. The results demonstrate that solution 4 prolonged the preparation cycle of the CAR-T cells, and the cell viability after cryopreservation and recovery was obviously reduced.

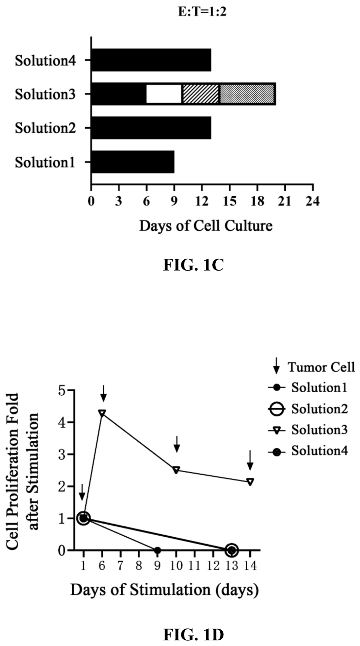

Further, the CD19-targeting CAR-T cells (CD19-targeting CAR-T cells as in patent 201710613317.6) prepared using the 4 solutions in the above were co-cultured with Nalm6-Luc-GFP tumor cells, and subjected to multiple repeated tumor killing assays. The main implementation process was as follows: co-culturing the CAR-T cells of 4 groups respectively with the Nalm6-Luc-GFP cells at the effector-to-target ratio of 1:2, detecting the proportion of GFP+ tumor cells in the cell population by flow cytometry, when the proportion of the GFP+ cells was below 10%, collecting and counting the cells, replenishing the tumor cells again at the effector-to-target ratio of 1:2 to perform repeated stimulation and killing, and taking a point at which the proportion of the GFP+ tumor cells in the cell population exceeded 90% as an endpoint of the killing assay. The number of rounds of tumor cell stimulation and the proliferation fold of the CAR-T cells during the stimulation process in each test group were calculated. The functionality and persistence of the CAR-T cells under different preparation solutions were reflected by the number of rounds of target cell stimulation on the CAR-T cells and proliferative capacity after the stimulation. The results are as shown in FIG. 1C, wherein the horizontal axis represents the number of days of sustained proliferation of the CAR-T cells *in vitro,* the vertical axis represents different preparation modes, and different column colors represent different numbers of rounds of stimulation. In FIG. 1D, the horizontal axis represents the number of days of sustained proliferation of the CAR-T cells *in vitro,* and the vertical axis represents proliferation of the cells from different preparation modes. The results demonstrate that solution 3 exhibited the optimal antitumor function, and exhibited a sustained antitumor function even at the low effector-to-target ratio of 1:8 (CAR-T:Nalm6).

Based on the above results, solution 3 is preferred for the preparation cycle of the CAR-T cells, that is:
Mononuclear cells obtained through isolation or T cells sorted from mononuclear cells (which may also be the above cells having undergone recovery after cryopreservation) were activated and cultured using 50 ng/ml anti-CD3 antibody (Recombinant anti-Human CD3 mAb, GMP-A018) in the system containing 5 ng/ml IL-7 and 25 ng/ml IL-21 cytokines. 12-24 hours after the activation, lentivirus containing the CD19-targeting CAR gene was added to perform CAR gene transduction. After adding the virus, culture was further performed for 14-28 h in the system containing 5 ng/ml IL-7 and 25 ng/ml IL-21 cytokines, and the CAR-T cells were harvested. The entire preparation cycle was 26 hours-52 hours. The CAR-T cells were CD19-targeting CAR-T cells, with a specific anti-CD19ScFv-8h-8TM-BBZ structure (CD19-targeting CAR), and sequences as shown in the Sequence Listing.

### 1.2 Study of Optimal Time Ranges of Cell Activation and Viral Transduction

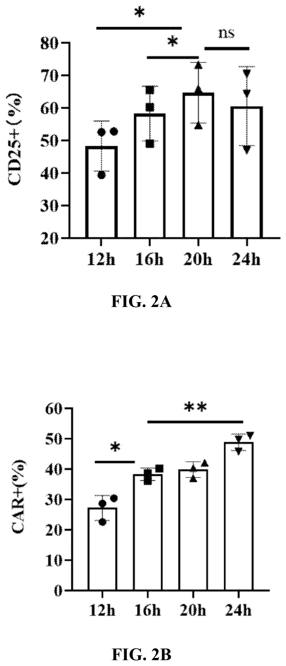

The T cells were activated using 50 ng/ml anti-CD3 antibody (Recombinant anti-Human CD3 mAb, GMP-A018), and cultured in the system containing 5 ng/ml IL-7 and 25 ng/ml IL-21 cytokines. After activation of 12 h, 16 h, 20 h, and 24 h, respectively, the lentiviral transduction was performed. Cells were harvested 18-28 hours following the viral transduction. The time range of activation for the prepared CAR-T cells was studied. Herein, the functionality of CAR-T was indirectly reflected by the activation intensity of CAR-T. CD25, as α chain of IL-2 receptor, was upregulated within 24 hours after the T-cell activation and remained elevated within several days. CD25 can be used to reflect the activation degree of the CAR-T cells. The harvested CAR-T cells were labelled with the anti-CD25 antibodies (CD25 BV421, Biolegend, 302630) and subjected to flow cytometry. The results were shown in FIG. 2A, wherein in FIG. 2A, the horizontal axis represents different cell activation time, and the vertical axis represents the CD25 expression positive rates of the CAR-T cells. The results demonstrate that expression of the cell activation marker CD25 tended to be upregulated with the activation time, reaching the optimal activation intensity at 20 h.

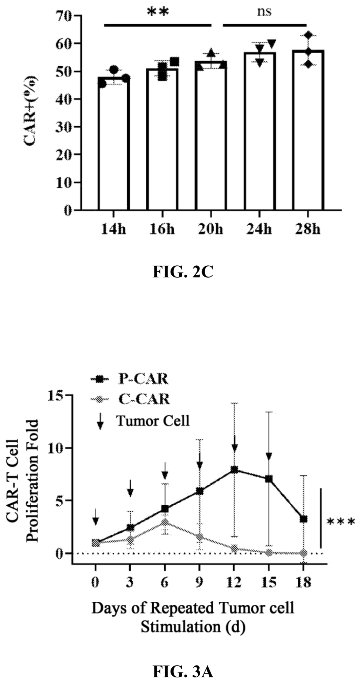

For the short-duration preparation, as the CAR-T cells were cultured for a relatively short period of time, and the T cells were in contact with the virus for a relatively short period of time, the CAR positive rate was usually relatively low in the short-duration preparation process, and the CAR positive rate of the CAR-T prepared with different time is also one of the parameters investigated for optimal time of cell activation and transduction. The results are as shown in FIG. 2B and FIG. 2C. FIG. 2B shows the CAR expression positive rates of the CAR-T cells prepared with different activation time, wherein the horizontal axis represents the different cell activation time, and the vertical axis represents the CAR expression positive rates. The results of the CAR positive rates of the cells demonstrate that the CAR positive rate tended to increase with the activation time, and the group with 24-hour activation maintained a higher proportion of CAR+ cells. FIG. 2C shows the CAR expression of the cells activated for 12-24 hours and harvested at various time after the viral transduction, wherein the horizontal axis represents different cell harvest time after the viral transduction, and the vertical axis represents the CAR expression positive rates. The results demonstrate that the CAR positive rate tended to increase with the activation time, reaching the highest positive rate at 20-28 h and remaining stable.

Based on the above results, the optimal T-cell activation time was 20 h-24 h, and the optimal cell harvest time after the viral transduction was 20 h-28 h.

### Example 2: Validation of Antitumor Efficacy of CAR-T Cells Prepared by Preparation Process of Present invention and CAR-T Cells Prepared by Conventional Process

### Preparation of CAR-T by Conventional Preparation Process: C-CAR

T cells from the donor PBMCs were activated using the anti-CD3/CD28 antibodies or magnetic beads at a bead-to-cell ratio of 1:1 (anti-CD3/CD28 Dynabeads 40203D). 12-24 hours after the activation, the cells were transduced with the CD19-targeting CAR lentiviral vector, and expanded for 8-11 days in the culture system containing only cytokines 5 ng/ml IL-7 and 25 ng/ml IL-21 (SCGM serum-free culture medium, CellGenix, 20802-0500). The CAR-T cells were harvested and cryopreserved.

### Preparation of CAR-T Cells by Process of Present invention: P-CAR

T cells from the donor PBMCs were activated using the 50 ng/ml CD3 antibodies (T cells were not in contact with CD28 antibodies throughout the process) (Recombinant anti-Human CD3 mAb, GMP-A018). 12-24 hours after the activation, the cells were transduced with the CD19-targeting CAR lentiviral vector, wherein the culture system was added with only two cytokines-5 ng/ml IL-7 and 25 ng/ml IL-21, and the culture medium (SCGM serum-free culture medium, CellGenix, 20802-0500). 14-28 hours after the viral transduction, the CAR-T cells were collected and cryopreserved. The CAR-T cells were CD19-targeting CAR-T cells, with specific sequences as shown in the Sequence Listing.

Control-T cells were prepared by the same process, without the viral transduction to serve as the control.

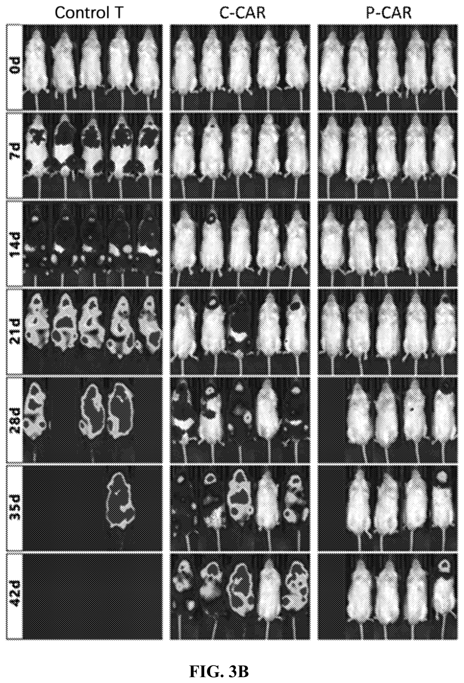

Female NOG mice aged 6-8 weeks were intravenously inoculated with Nalm6-Luc-GFP tumor cells at 1.00E+06 per mouse. Three days after the tumor inoculation, the CAR-T cells prepared by two solutions were administered intravenously at the dose of 1.00E+06 CAR+ per mouse. The *in vivo* antitumor efficacy of the CAR-T cells was compared weekly via live animal imaging. At the same time, the CAR-T cells prepared by the two preparation processes were co-cultured with Nalm6-Luc-GFP cells at the effector-to-target ratio of 1:1, the tumor cells were added repeatedly every 3 days at the same effector-to-target ratio for stimulation, and the proliferation of the CAR-T cells was calculated by counting cells. The persistence results of the CAR-T cells prepared by different processes in the tumor-bearing mice are shown in FIG. 3A, wherein in FIG. 3A the horizontal axis represents the time points of CAR-T cell detection, and the vertical axis represents the proliferation folds of the CAR-T cells. The results demonstrate that the rapid preparation process P-CAR exhibited superior cell expansion capacity compared with the conventional process C-CAR upon multiple rounds of tumor antigen stimulation. The efficacy results of the CAR-T cells prepared by the two processes in the tumor-bearing mice are shown in FIG. 3B. The *in vivo* animal experimental results demonstrate that the antitumor functionality of the P-CAR-T cells was significantly superior to that of the CAR-T cells prepared by the conventional process.

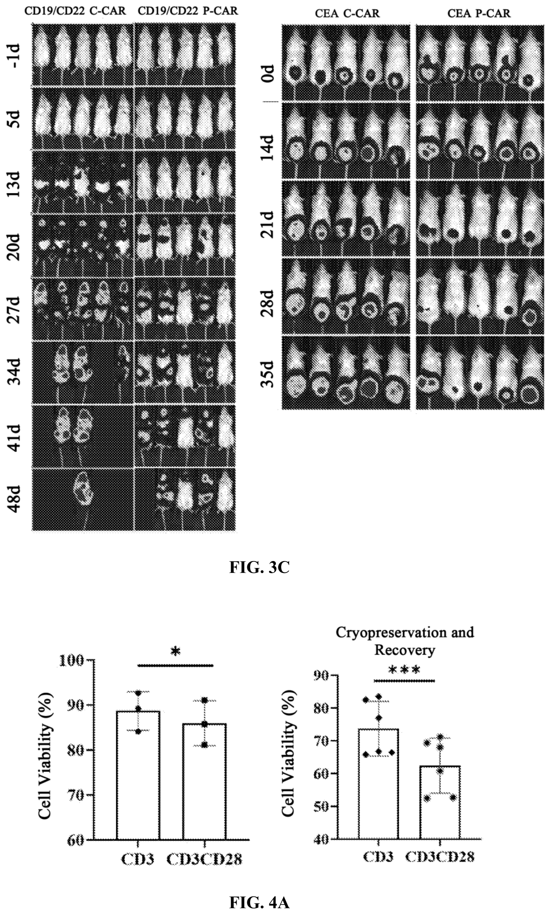

Feasibility of the CAR-T cells of the present invention in a variety of target CAR-T cell production processes was further validated. P-CAR and C-CAR were prepared according to the above solutions, and transduced with the CD19/CD22-targeting bispecific lentiviral vector and the CEA-targeting lentiviral vector. When comparing the *in vivo* functionalities of the CD19/CD22-targeting P-CAR and C-CAR, female NOG mice aged 6-8 weeks were intravenously inoculated with Nalm6-Luc-GFP tumor cells at 1.00E+06 per mouse. Three days after the tumor inoculation, the CAR-T cells prepared by two solutions were administered intravenously at the dose of 1.00E+06 CAR+ per mouse. The *in vivo* antitumor efficacy of the CAR-T cells was compared weekly via live animal imaging. When comparing the *in vivo* functionalities of CEA-targeting P-CAR and C-CAR, female NOG mice aged 6-8 weeks were intravenously inoculated with CEA-overexpressing DLD1-luc-GFP tumor cells at the dose of 1.00E+06 CAR+ per mouse, establishing the subcutaneous solid tumor models. Two weeks after the tumor inoculation, the CAR-T cells prepared by the two solutions were injected intravenously at 5.00E+05 CAR+ per mouse. The *in vivo* antitumor efficacy of the CAR-T cells was compared weekly via live animal imaging. The efficacy results of the CAR-T cells targeting CD19/CD22 or CEA in the tumor-bearing mice are shown in FIG. 3C. The *in vivo* animal experimental results demonstrate that the antitumor functionality of the P-CAR-T cells targeting CD19/CD22 or CEA was significantly superior to that of the CAR-T cells prepared by the conventional process. All of the CAR-T cells of the process of the present invention demonstrated superior *in vivo* antitumor efficacy in multiple-target tumor models to those by the conventional process. The CAR sequences targeting CD19/CD22 or CAE are shown in the Sequence Listing, respectively.

### Example 3 Comparison between anti-CD3 antibody Activation Solution and Anti-CD3/CD28 Magnetic Bead Activation Solution

The CAR-T cells of the process of the present invention were prepared according to the solution of P-CAR-T in Example 2, denoted by CD3. The T cells were activated using anti-CD3/CD28 magnetic beads at the bead-to-cell ratio of 1:1 (anti-CD3/CD28 Dynabeads, 40203D), followed by lentiviral transduction after 12-24 hours. The cells were cultured in the system containing different cytokines 5 ng/ml IL-7 and 25 ng/ml IL-21 (SCGM serum-free culture medium, CellGenix, 20802-0500). 14-28 hours after the viral transduction, the cell harvest was completed, obtaining the CAR-T cells activated with the anti-CD3/CD28 beads, denoted by CD3/CD28. The CAR-T cells were CD19-targeting CAR-T cells, with specific sequences as shown in the Sequence Listing. The CAR-T cell viability after activation and after cryopreservation and recovery was assessed, and the results are as shown in FIG. 4A, wherein the horizontal axis represents two activation solutions, and the vertical axis represents the CAR-T cell viability. Anti-CD3 antibody activation maintained higher cell viability and higher viability stability of the CAR-T cells after cryopreservation and recovery.

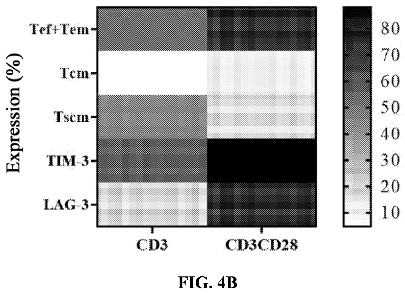

The CAR-T cells prepared by the two activation solutions were co-cultured with Nalm6-Luc-GFP cells at the effector-to-target ratio of 1:1 (CAR-T:Nalm6), and the phenotype of the CAR-T cells was detected after 3 times of repeated tumor stimulation. The results are shown in FIG. 4B, wherein the horizontal axis represents the two activation solutions, and the vertical axis represents different phenotypes of the CAR-T cells. The CAR-T cells prepared by the anti-CD3 antibody activation solution demonstrated a higher Tscm (T stem cell memory) phenotype, and the proportions of the TIM-3 and LAG-3 immuno-suppressive phenotypes were lower, indicating that the anti-CD3 antibody activation has the effects of maintaining the cell memory phenotype and reducing the cell exhaustion.

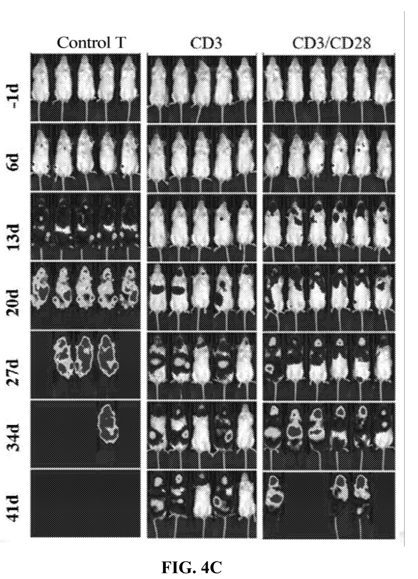

The *in vivo* efficacy of the anti-CD3 antibody activation solution and the anti-CD3/CD28 bead activation solution was further validated. Female NOG mice aged 8-10 weeks were intravenously inoculated with Nalm6-Luc-GFP tumor cells at 1.00E+06 per mouse. Three days after the tumor inoculation, the CAR-T cells prepared by two solutions were administered intravenously at 1.00E+06 CAR+ per mouse. The *in vivo* antitumor efficacy of the CAR-T cells was compared, and the survival of the mice in the two solutions was evaluated. The results are shown in FIG. 4C. The mice injected with the CAR-T cells in the CD3/CD28 activation solution began to die by day 41, and superior antitumor efficacy was not exhibited in the mice. The short-duration CAR-T preparation solution of the present invention, which used the anti-CD3 antibody activation alone, demonstrated higher safety and superior *in vivo* efficacy.

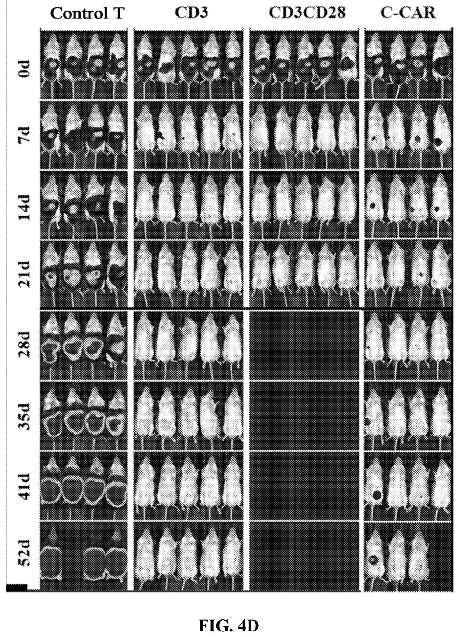

Further, the *in vivo* efficacy and safety of the P-CAR solution in the present invention were evaluated with the short-duration solution (CD3/CD28) with the anti-CD3/CD28 magnetic bead activation used in the art and the CD70-targeting CAR-T cells prepared by the conventional C-CAR process (CAR structure was incorporated with the CD70 CAR sequence of patent 202111239047.X). SKOV3-Luc-GFP tumor cells were intraperitoneally inoculated into the mice at 1.00E+06 per mouse. Seven days after the tumor inoculation, the CAR-T cells prepared by three solutions were intraperitoneally administered at the dose of 3.00E+05 CAR+ per mouse. The *in vivo* antitumor efficacy of the CAR-T cells was compared, and survival of the mice was evaluated across the three solutions. The results are as shown in FIG. 4D. Although the CAR-T cells in the CD3/CD28 solution were superior to the CAR-T cells in the C-CAR solution, all mice died by day 28 due to excessive immune response *in vivo.* However, the P-CAR solution in the present invention not only demonstrated *in vivo* efficacy superior to that of the C-CAR and comparable to that of the CD3/CD28 solution, but also was significantly superior to the CD3/CD28 solution in safety. The short-duration CAR-T preparation solution of the present invention, which used the anti-CD3 antibody activation alone, demonstrated higher safety and superior *in vivo* efficacy.

### Example 4 Application of Different Cytokines or Combinations thereof in Short-Duration CAR-T Preparation Solution

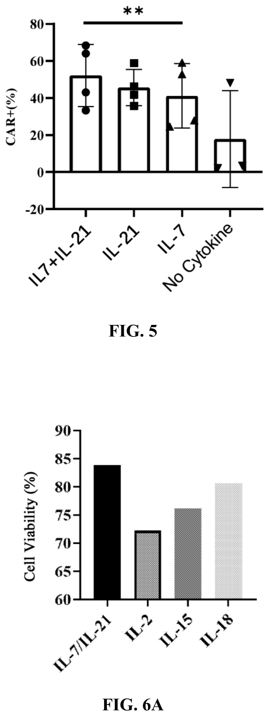

T cells were activated with 50 ng/ml anti-CD3 antibody (Recombinant anti-Human CD3 mAb, GMP-A018) for 12-24 hours, followed by lentiviral transduction. The cells were cultured in systems respectively as follows: containing both 5 ng/ml IL-7 and 25 ng/ml IL-21 cytokines, respectively containing 5 ng/ml IL-7 and 25 ng/ml IL-21, and no-cytokine throughout the process (SCGM serum-free culture medium, CellGenix, 20802-0500). The cell harvest was completed 14-28 hours after the viral transduction. The cell harvest was completed the next day following the viral transduction, and different cytokine strategies were compared. The cell CAR positive rate from the transduction under the no-cytokine condition throughout the process was compared. The CAR positive rate was labeled by PE-PL and then subjected to flow cytometry. The results are as shown in FIG. 5, wherein the horizontal axis represents different cytokine solutions, and the vertical axis represents the CAR expression positive rates. The results demonstrate that, compared with no-cytokine or single-cytokine culture, incorporation of two cytokines IL-7 and IL-21 to the short-duration preparation process of the present invention enhanced the CAR positive rate.

Further, four groups of solutions were designed for different cytokines:
Solution 1: 50 ng/ml anti-CD3 antibody for activation, and 5 ng/ml IL-7 and 25 ng/ml IL-21 cytokine for culture;
Solution 2: 50 ng/ml anti-CD3 antibody for activation, and 500 IU/ml IL-2 cytokine for culture;
Solution 3: 50 ng/ml anti-CD3 antibody for activation, and 20 ng/ml IL-15 cytokine for culture; and
Solution 4: 50 ng/ml anti-CD3 antibody for activation, and 50 ng/ml IL-18 cytokine for culture.

Using the P-CAR preparation solution of Example 2, the "culture system (SCGM serum-free culture medium, CellGenix, 20802-0500) added with cytokines 5 ng/ml IL-7 and 25 ng/ml IL-21" was replaced with the cytokine solutions of the above solutions 1-4, and the prepared CAR-T cells were respectively denoted by IL-7/IL-21, IL-2, IL-15, and IL-18. The cells prepared in the above were harvested and then cryopreserved using the commercial cryopreservation solution.

### 4.1 Post-Recovery Viability Assay of Cryopreserved CAR-T Cells

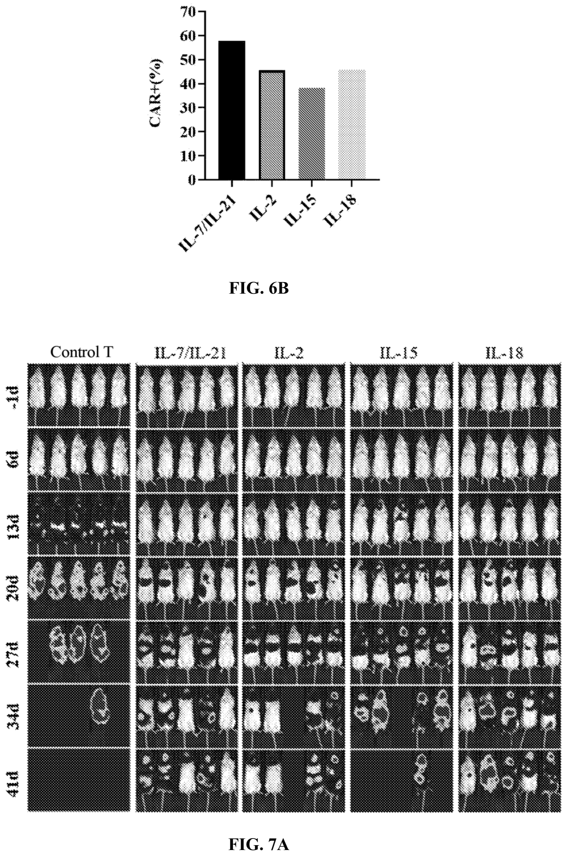

After the cryopreserved CAR-T cells were rapidly recovered in the water bath at 37 °C, a certain amount of the cells were subjected to the cell viability assay using AOPI, and the detection results are as shown in FIG. 6A, wherein the horizontal axis represents the CAR-T cells from different preparation solutions, and the vertical axis represents the proportions of viable CAR-T cells. The post-recovery CAR-T cells were further subjected to CAR expression detection using the solution of Example 1, as shown in FIG. 6B, and the results demonstrated that the cytokine combination of IL-7 + IL-21 maintained higher CAR positive rate and higher cell viability after recovery.

### 4.2 In vivo Efficacy Validation

Female NOG mice aged 8-10 weeks were intravenously inoculated with Nalm6-Luc-GFP tumor cells at 1.00E+06 per mouse. Three days after the tumor inoculation, the CAR-T cells prepared by four solutions were administered intravenously at 1.00E+06 CAR+ per mouse. The *in vivo* antitumor efficacy of the CAR-T cells was compared, and the survival of the mice in the two solutions was evaluated. The CAR-T cells were CD19/CD22-targeting CAR-T cells, with specific sequences as shown in the Sequence Listing.

The results are as shown in FIG. 7A. The IL-7 + IL-21 culture solution demonstrated superior antitumor efficacy in the mice compared with the IL-15 and IL-18 solutions, and at the same time, exhibited the same *in vivo* efficacy as the IL-2 solution. However, in the culture solutions of IL-2 and IL-15, the death of mice started to be monitored by 34 days after the CAR-T cell infusion, exhibiting higher safety-related risk than that of the IL-7 + IL-21 solution.

Further, for solution 1, solution 2, solution 4, the combination of 50 ng/ml anti-CD3 antibody for activation and 5 ng/ml IL-7, and the combination of 50 ng/ml anti-CD3 antibody for activation and 25 ng/ml IL-21, safety comparison was conducted using the P-CAR process and the existing CD3/CD28 short-duration preparation solution in the art in Example 3.

In the existing CD3/CD28 short-duration preparation solution, the T cells were activated using the anti-CD3/CD28 magnetic beads, followed by lentiviral transduction, and culture in the system containing 100 IU/ml-500 IU/ml IL-2, denoted as CD3CD28 + IL-2 solution. In the following example validation, the specific existing CD3/CD28 short-duration preparation solution in Example 3 was adopted, denoted as CD3CD28, or the solution with the cytokine component IL-2 was adopted. IL-2 concentration was 500 IU/ml in the CD3CD28 + IL-2 solution.

Cytokine release syndrome (CRS) is an acute systemic inflammatory syndrome featured by fever and dysfunction of multiple organs, and is related to the chimeric antigen receptor (CAR) T cell treatment, the therapeutic antibody, and haploid allogeneic transplantation. Based on the clinical literature *"*Side-effect management of chimeric antigen receptor (CAR) T-cell treatment" published in 2020, which summarizes the clinical adverse effects following the CAR-T cell treatment, the section on "Cytokine release syndrome" states that "As a supraphysiologic inflammatory state, CRS (Figure 1) is triggered by inflammatory cytokines and chemokines released by CAR T cells, e.g. interferon (IFN) γ, tumor necrosis factor (TNF) α, granulocyte macrophage colony-stimulating factor (GM-CSF), interleukin (IL)-2, IL-8 and IL-10, after engaging with the corresponding target antigen." It can be seen therefrom that in the CAR-T cell treatment, CRS is primarily caused by excessive or uncontrolled release of pro-inflammatory cytokines such as IL-2, TNF-α, and IFN-γ following the activation of the CAR-T cells by the tumor cells. These pro-inflammatory cytokines are markers of CAR-T functionality, but must be maintained within an appropriate concentration range and prevented from excessive or uncontrolled release. Therefore, provided that killing efficacy on the tumor cells is validated, lower secretion release of cytokines IL-2, TNF-α, and IFN-γ is safer.

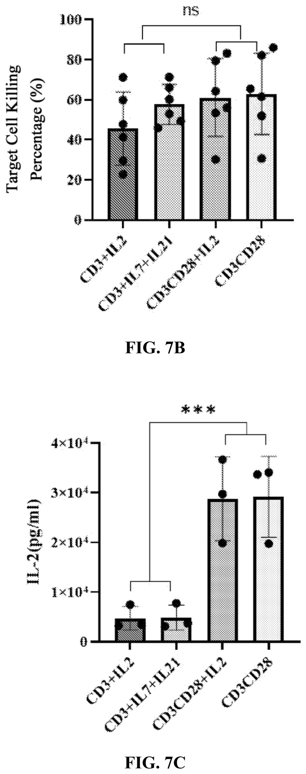

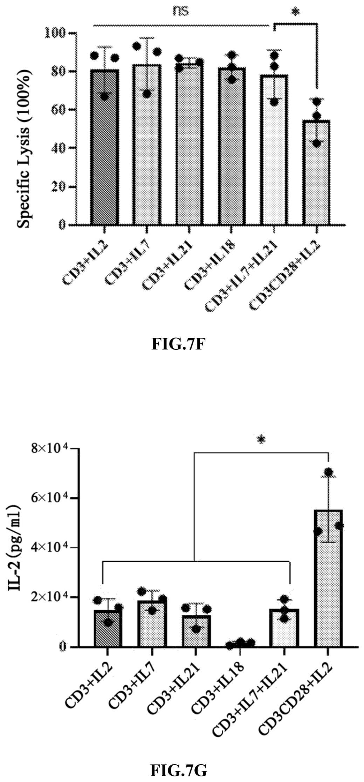

Therefore, safety of the P-CAR process and the CD3/CD28 short-duration preparation processes (CD3CD28 + IL-2 solution, CD3CD28 solution) was evaluated through a combination of both experiments of *in vitro* killing and cytokine secretion detection. CAR-T cells were the preceding CD19-targeting CAR-T, and CD19/CD22-targeting CAR-T cells. Tumor target cells were Nalm6-Luc-GFP. The effector-to-target ratio was 1:1. Tumor cells and CAR-T cells were co-incubated for 24 hours, followed by analysis of the killing capacity of the CAR-T cells on the tumor cells, respectively. Results were shown in FIG. 7B and FIG. 7F, wherein FIG. 7B shows efficacy results of the CD19/CD22-targeting CAR-T cells prepared through different processes, and FIG. 7F shows efficacy results of the CD19-targeting CAR-T cells prepared through different processes. Both P-CAR process and CD3/CD28 short-duration preparation process had an effective capability of killing Nalm6-Luc-GFP, and the functionality of the CAR-T cells prepared through the P-CAR process was not inferior to (or comparable to) that of the CD3/CD28 short-duration preparation process. For some targets, the efficacy of the P-CAR process was superior to that of the CD3/CD28 short-duration preparation process.

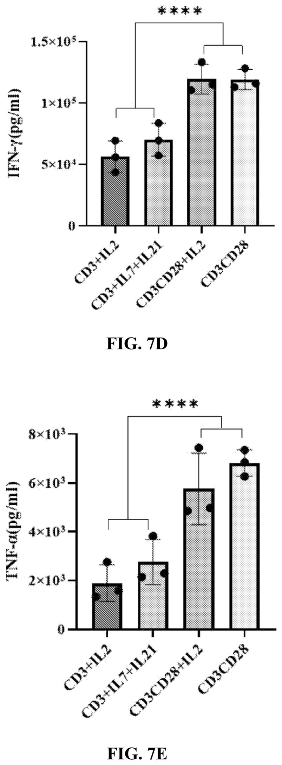

Killing supernatant thereof was further aspirated to detect the cytokine secretion of IL-2 (Invitrogen, Cat. No. 88-7025-88), TNF-α (Biolegend, Cat. No. 430201), and IFN-γ (BD, Cat. No. 555142) by ELISA (enzyme-linked immunosorbent assay). A specific detection method was operated according to the instructions. Cytokine secretion results are as shown in FIGS. 7C-7G, wherein FIGS. 7C-7E show cytokine secretion after tumor cell killing by CD19/CD22-targeting CAR-T cells prepared through different processes: FIG. 7C shows secretion results of IL-2 from different processes, FIG. 7D shows secretion results of IFN-γ from different processes, FIG. 7E shows secretion results of TNF-αfrom different processes, and FIG. 7G shows cytokine secretion after tumor cell killing by CD19-targeting CAR-T cells prepared through different processes. The results demonstrate that the secretion of cytokines IL-2, TNF-α, and IFN-γ by P-CAR was greater than 500 pg/ml, and along with the *in vitro* killing capability, it is indicated that the CAR-T from the P-CAR process has potent tumor cell killing capability, and the secretion of cytokines IL-2, TNF-α, and IFN-γ by P-CAR was significantly lower than those of the CD3/CD28 short-duration preparation process, indicating that the CAR-T prepared by the P-CAR process has significantly better safety than that from the CD3/CD28 short-duration preparation process.

Specific killing percentages corresponding to FIG. 7B are listed in Table 2 below.

**Table 2**

| CD3 + IL2 | CD3 + IL7 + IL21 | CD3CD28 + IL2 | CD3CD28 |
|---|---|---|---|
| 41.37 | 66.08 | 83.19 | 86 |
| 22.9 | 46.08 | 64.36 | 65.43 |
| 29.71 | 52.95 | 79.58 | 82.21 |
| 71.31 | 71.4 | 56.09 | 61.59 |
| 47.97 | 49.36 | 30.27 | 30.81 |
| 59.99 | 60.27 | 53.46 | 52.06 |

Specific IL-2 cytokine secretion values (pg/ml) corresponding to FIG. 7C are listed in Table 3 below.

**Table 3**

| CD3 + IL2 | CD3 + IL7 + IL21 | CD3CD28 + IL2 | CD3CD28 |
|---|---|---|---|
| 3438.787 | 3704.097 | 29754.965 | 33680.212 |
| 3255.645 | 3116.233 | 19871.141 | 19756.75 |
| 7469.542 | 7744.703 | 36672.248 | 34070.886 |

Specific IFN-γ cytokine secretion values (pg/ml) corresponding to FIG. 7D are listed in Table 4 below.

**Table 4**

| CD3 + IL2 | CD3 + IL7 + IL21 | CD3CD28 + IL2 | CD3CD28 |
|---|---|---|---|
| 43665.676 | 57316.831 | 110462.29 | 112990.093 |
| 55931.144 | 69555.139 | 114885.37 | 115941.599 |
| 69403.195 | 83740.451 | 133135.215 | 128360.003 |

Specific TNF-α cytokine secretion values (pg/ml) corresponding to FIG. 7E are listed in Table 5 below.

**Table 5**

| CD3 + IL2 | CD3 + IL7 + IL21 | CD3CD28 + IL2 | CD3CD28 |
|---|---|---|---|
| 1335.03 | 2152.444 | 4978.401 | 6846.206 |
| 1583.939 | 2291.187 | 4848.312 | 6261.967 |
| 2753.558 | 3820.563 | 7441.971 | 7347.821 |

Specific killing percentages corresponding to FIG. 7F are listed in Table 6 below.

**Table 6**

| CD3 + IL2 | CD3 + IL7 | CD3 + IL21 | CD3 + IL7 + IL21 | CD3 + IL18 | CD3CD28 + IL2 |
|---|---|---|---|---|---|
| 67 | 68.37 | 81.68 | 64.24 | 88.63 | 42.66 |
| 87.24 | 90.34 | 84.93 | 82.83 | 82.46 | 64.38 |
| 88.37 | 93.27 | 86.92 | 88.37 | 75.81 | 56.92 |

Specific IL-2 cytokine secretion values (pg/ml) corresponding to FIG. 7G are listed in Table 7 below.

**Table 7**

| CD3 + IL2 | CD3 + IL7 | CD3 + IL21 | CD3 + IL7 + IL21 | CD3 + IL18 | CD3CD28 + IL2 |
|---|---|---|---|---|---|
| 18818.203 | 19344.739 | 15244.384 | 14773.989 | 2074.481 | 49047.152 |
| 15887.119 | 22440.545 | 15833.996 | 19314.074 | 1747.662 | 70638.768 |
| 9868.311 | 14666.943 | 7235.675 | 11466.374 | 557.109 | 46652.491 |

The results demonstrate that through comparion between the activation solution using the anti-CD3 antibody alone and the activation solution using the anti-CD3/CD28 antibody or the magnetic bead coated with the anti-CD3/CD28 antibody, the T-cell activation signal provided by the anti-CD3 antibody can reduce the risk of adverse effects while maintaining the potent antitumor functionality of the short-duration CAR-T cells. By means of the CD3 activation solution in combination with the cytokines such as IL7, IL-21, IL7 + IL-21, IL2, IL15, and IL-18, the results demonstrate that the cytokine combination solution of IL-7 + IL-21 can maintain a more stable and higher CAR positive rate, and at the same time, exhibit a better therapeutic effect in animal models and maintain the survival of mice. To sum up, the anti-CD3 antibody activation coupled with IL-7 + IL-21 cytokine combination demonstrates more prominent application feasibility on the short-duration CAR-T cell platform.

### Example 5 Human Drug Safety of CAR-T Cells Prepared in Short Duration in the Present invention

In order to further confirm the clinical treatment safety and efficacy of CAR-T cells P-CAR-T prepared through the short-duration preparation solution of the present invention, T cells were activated with 50 ng/ml anti-CD3 antibody and cultured with 5 ng/ml IL-7 + 25 ng/ml IL-21 cytokines in the SCGM serum-free culture medium (CellGenix, 20802-0500). The T cells were activated for 12-24 h, followed by transduction with the CD19-targeting lentiviral vector. After the viral transduction, the CD19-P-CAR-T cells were harvested at 14-28 h, and treatment and efficacy evaluation were completed for 19 patients with acute lymphoblastic leukemia and lymphoma. A comparison was made between clinical outcomes of the CD19-Fast CAR-T cells, prepared within two days after PBMC isolation using the rapid preparation solution with anti-CD3/CD28 antibody-coated Dynabeads and IL-2, for the treatment of acute lymphoblastic leukemia in the literatures "Novel CD19 chimeric antigen receptor T cells manufactured next-day for acute lymphoblastic leukemia" (literature 1) and "Next-day manufacture of a novel anti-CD19 CAR-T therapy for B-cell acute lymphoblastic leukemia: first-in-human clinical study" (literature 2), and the results are listed in Table 8 below.

**Table 8 In vivo Efficacy and Safety Comparison of Short-Duration CAR-T Cells Prepared by Different Solutions**

| Group | Patient (case) | CR (%) | Total CRS (%) | CRS (%) (≥ grade 3) | Total iCANS (%) | iCANS (%) (grade 1-2) | iCANS (%) (≥ grade 3) |
|---|---|---|---|---|---|---|---|
| Literature 2 | 25 | 92.0% | 96.0% | 24.0% | 28.0% | 0% | 28.0% |
| Literature 1 | 21 | 94.4% | 95.2% | 52.4% | 28.6% | 28.6% | |
| P-CAR-T | 19 | 100% | 94.7% | 5.26% | 15.79% | 15.79% | 0% |

The CD19-targeting short-duration CAR-T cells prepared by the anti-CD3 antibody preparation solution of the present invention for the treatment of acute lymphoblastic leukemia and lymphoma, compared with the cells prepared by the anti-CD3/CD28 antibody-coated Dynabeads and IL-2 solutions, exhibited a higher clinical complete response (CR) rate, lower CRS and iCANS adverse effect rates, and significantly lower proportion of severe CRS and iCANS of grade 3 or higher. It is further proved that the solution using the anti-CD3 antibody + IL7/IL-21 for rapid preparation of the cell product exhibits a better clinical effect and a lower adverse effect rate than the solution of the anti-CD3/CD28 antibody + IL-2, and has a higher clinical application value.

### Example 6: anti-CD3 antibody Concentration Testing

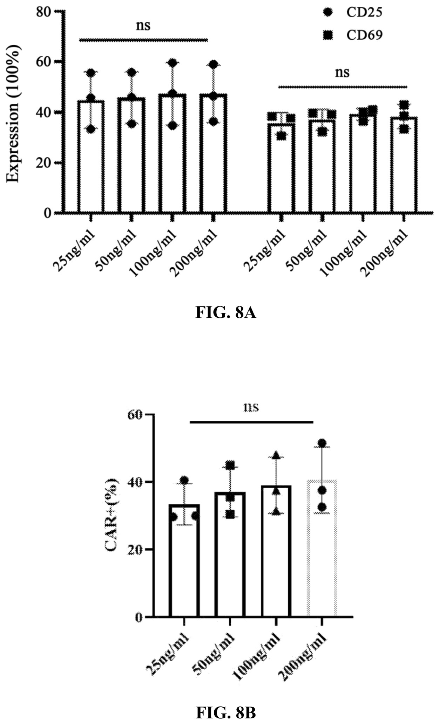

T cells were activated with 25-200 ng/ml anti-CD3 antibody (Recombinant anti-Human CD3 mAb, GMP-A018), and cultured in systems containing 5 ng/ml IL-7 and 25 ng/ml IL-21 cytokines (SCGM serum-free culture medium, CellGenix, 20802-0500) for 12 h-24 h respectively, followed by lentiviral transduction. The cells were harvested 18-24 hours after the viral transduction. The effect of different anti-CD3 antibody concentrations on the preparation process was investigated. Appropriate concentrations of the anti-CD3 antibody were tested by detecting the ratios of cell activation indexes CD25/CD69 and CAR+ cells. The results are shown in FIG. 8A and FIG. 8B, wherein the horizontal axis represents the culture systems at different anti-CD3 antibody concentrations with 5 ng/ml IL-7 and 25 ng/ml IL-21 cytokines, and the vertical axis represents the cell activation percentages and the CAR expression positive rates, respectively. The results demonstrate that activation of the CAR-T cells and the CAR gene transduction can be well completed at the use concentration ranging from 25 ng/ml to 200 ng/ml.

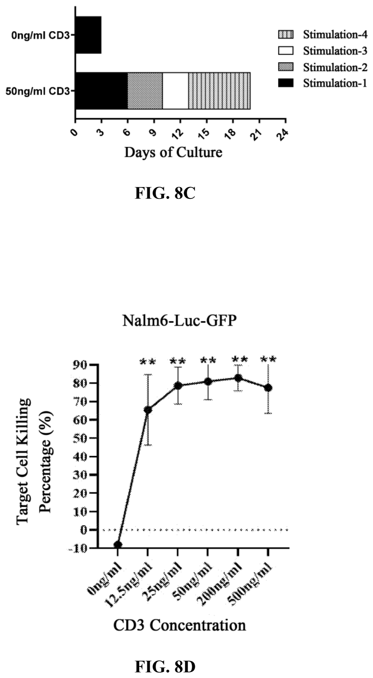

At the same time, the *in vitro* antitumor functionality of the CAR-T cells prepared by the process of the present invention was also confirmed through multiple sustained rounds of tumor cell stimulation experiments *in vitro,* at the anti-CD3 antibody concentrations of 0 ng/ml, 25 ng/ml, 50 ng/ml, 200 ng/ml, and 500 ng/ml. The T cells were activated with 0 ng/ml, 25 ng/ml, 50 ng/ml, 200 ng/ml, and 500 ng/ml anti-CD3 antibody (Recombinant anti-Human CD3 mAb, GMP-A018) for 12-24 h, followed by transduction with CD19-targeting lentiviral vector, and culture in the system containing both 5 ng/ml IL-7 and 25 ng/ml IL-21 cytokines (SCGM serum-free culture medium, Cell Genix, 20802-0500). The cell harvest was completed 14-28 hours after the viral transduction. CD19-targeting CAR-T cells (CD19-targeting CAR-T cells as in patent 201710613317.6) prepared using the above solution were co-cultured with Nalm6-Luc-GFP tumor cells at the effector-to-target ratio of 1:2, and subjected to multiple repeated tumor killing assays. The proportion of GFP+ tumor cells in the cell population was detected by flow cytometry. When the proportion of the GFP+ cells was below 10%, the cells were collected and counted, and the tumor cells were replenished again at the effector-to-target ratio of 1:2 to perform repeated stimulation and killing. The point at which the proportion of the GFP+ tumor cells in the cell population exceeded 90% was taken as an endpoint of the killing assay. The number of rounds of tumor cell stimulation and the proliferation fold of CAR-T cells during the stimulation process in each test group were calculated. The functionality and persistence of the CAR-T cells under different preparation solutions were reflected by the number of rounds of target cell stimulation on the CAR-T cells and proliferative capacity after the stimulation. The results are shown in FIG. 8C. In the absence of anti-CD3 antibody, that is, with 0 ng/ml anti-CD3 antibody, CAR-T cells failed to be activated or proliferated upon stimulation by the target cells. Therefore, the short-duration CAR-T cell preparation process in the present invention can be well used when the anti-CD3 antibody concentration ranges from 25 ng/ml to 200 ng/ml.

Further, the T cells were activated with 0 ng/ml, 12.5 ng/ml, 25 ng/ml, 50 ng/ml, 200 ng/ml, and 500 ng/ml anti-CD3 antibody (Recombinant anti-Human CD3 mAb, GMP-A018) for 12-24 h, followed by transduction with CD19-targeting lentiviral vector, and culture in the system containing both 5 ng/ml IL-7 and 25 ng/ml IL-21 cytokines (SCGM serum-free culture medium, Cell Genix, 20802-0500). The cell harvest was completed 14-28 hours after the viral transduction. CD19-targeting CAR-T cells (CD19-targeting CAR-T cells as in patent 201710613317.6) prepared using the above solution were co-cultured with Nalm6-Luc-GFP tumor cells at the effector-to-target ratio of 1:1, and the killing capability of the prepared CAR-T cells was confirmed, wherein the results are shown in FIG. 8D, and under the culture solutions with 12.5 ng/ml, 25 ng/ml, 50 ng/ml, 200 ng/ml, and 500 ng/ml anti-CD3 antibody, they all exhibited significant killing capability, and the killing percentages are listed in Table 9 below.

**Table 9 Tumor Cell Killing Percentage of CAR-T Cells Prepared by P-CAR Process at Varying CD3 Concentrations**

| 0 ng/ml | 12.5 ng/ml | 25 ng/ml | 50 ng/ml | 200 ng/ml | 500 ng/ml |
|---|---|---|---|---|---|
| -8.16 | 78.99 | 85.8 | 88.08 | 87.79 | 87.48 |
| -7.9 | 51.92 | 71.65 | 73.89 | 77.88 | 67.6 |

The results demonstrate that the short-duration CAR-T cell preparation process in the present invention can be well used when the anti-CD3 antibody concentration ranges from 12.5 ng/ml to 200 ng/ml.

### Example 7 IL-7/IL-21 Cytokine Concentration Testing

During the preparation process of P-CAR-T cells, T cells were activated for 12-24 h, followed by viral transduction. The cells were harvested 14-28 h after the viral transduction. The T cells were activated using 50 ng/ml anti-CD3 antibody (Recombinant anti-Human CD3 mAb, GMP-A018), and cultured using cytokines IL-7 and IL-21 combined at different concentrations in SCGM serum-free culture medium (CellGenix, 20802-0500). Effects of different cytokine concentrations on maintaining the cell viability and viral transduction efficiency were evaluated.
Solution 1: 2.5 ng/ml IL-7 and 12.5 ng/ml IL-21
Solution 2: 5 ng/ml IL-7 and 25 ng/ml IL-21
Solution 3: 10 ng/ml IL-7 and 50 ng/ml IL-21
Solution 4: 2.5 ng/ml IL-7 and 50 ng/ml IL-21
Solution 5: 10 ng/ml IL-7 and 12.5 ng/ml IL-21

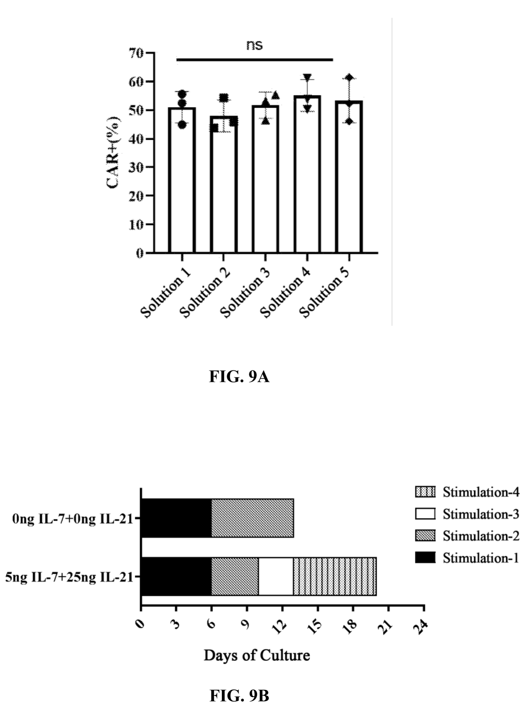

Selection of different cytokine concentrations was investigated by detecting the ratio of CAR+ cells, and the results are as shown in FIG. 9A. The combination of 2.5-10 ng/ml IL-7 and 12.5-50 ng/ml IL-21 cytokines can well complete the CAR gene transduction of CAR-T cells.

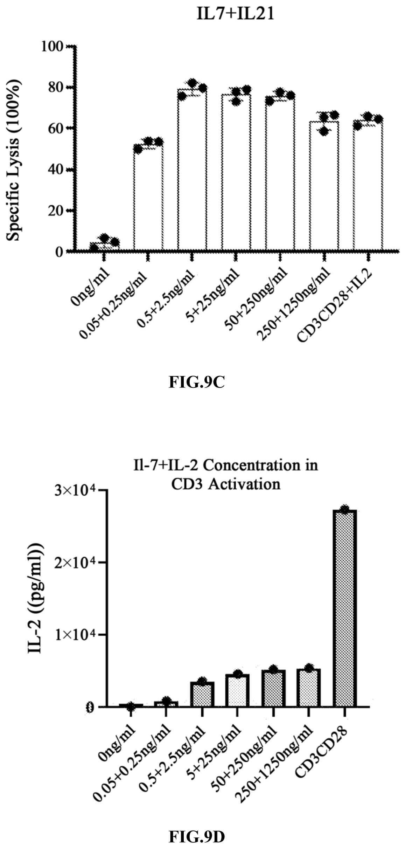

Further, using combinations of 0.05 ng/ml IL-7 and 2.5 ng/ml IL-21, 0.5 ng/ml IL-7 and 2.5 ng/ml IL-21, 5 ng/ml IL-7 and 25 ng/ml IL-21, 50 ng/ml IL-7 and 250 ng/ml IL-21, and 250 ng/ml IL-7 and 1250 ng/ml IL-21, killing and cytokines of the P-CAR short-duration preparation solution and the prior art CD3/CD28 short-duration preparation solution were compared. The solution of Example 3 was used as the CD3/CD28 short-duration preparation solution, and the results are as shown in FIG. 9C and FIG. 9D. FIG. 9C compares tumor cell killing between the P-CAR short-duration preparation solution and the prior art CD3/CD28 short-duration preparation solution. The results demonstrate that the combination with IL-7 at concentration of 0.05-250 ng/ml and IL21 at concentration of 0.25-1250 ng/ml could exert a potent killing capability on the tumor cells, and the killing capability was not inferior to that of the existing CD3/CD28 short-duration preparation solution, with specific data are as listed in Table 10. FIG. 9D shows comparison of IL-2 cytokine secretion capability of the CAR-T cells from the P-CAR short-duration preparation solution and the prior art CD3/CD28 short-duration preparation solution after tumor cell activation, wherein the vertical axis represents the concentration of IL-2 secretion, the horizontal axis represents combinations of IL-7 and IL-21 cytokine at different concentrations, and the cytokine secretion concentrations are as listed in Table 11 below. The results demonstrate that the CAR-T cells prepared through the P-CAR short-duration preparation solution secreted IL-2 cytokine greater than 500 pg/ml upon the tumor cell activation, significantly less than that of the CAR-T prepared through the prior art CD3/CD28 short-duration preparation solution.

**Table 10 IL-2 Cytokine Secretion (pg/ml) by CAR-T Cells Prepared by P-CAR Process with Different IL-7 and IL-21 Concentration Combinations after Activation**

| 0 ng/ml | 0.05+0.25 ng/ml | 0.5+2.5 ng/ml | 5+25 ng/ml | 50+250 ng/ml | 250+1250 ng/ml | CD3CD28 |
|---|---|---|---|---|---|---|
| 101.35 | 885.444 | 3606.414 | 4643.141 | 5256.35 | 5433.289 | 27390.291 |

**Table 11 Killing Functionality of CAR-T Cells Prepared by P-CAR Process with Different IL-7 and IL-21 Concentration Combinations**

| 0 ng/ml | 0.05+0.25 ng/ml | 0.5+2.5 ng/ml | 5+25 ng/ml | 50+250 ng/ml | 250+1250 ng/ml | CD3CD28 |
|---|---|---|---|---|---|---|
| 6.8 | 53.59 | 79.67 | 78.99 | 73.25 | 58.67 | 66.05 |
| 1.83 | 53.88 | 82.13 | 77.85 | 76.15 | 66.59 | 64.63 |
| 4.89 | 49.86 | 75.83 | 73.23 | 77.82 | 65.65 | 61.29 |

Through combination of FIG. 9A, FIG. 9C, and FIG. 9D, in the P-CAR short-duration preparation solution of the present invention, the IL-7 cytokine concentration was 0.05-250 ng/ml, and the IL21 concentration was 0.25-1250 ng/ml.

At the same time, the *in vitro* antitumor functionality of the CAR-T cells prepared by the process of the present invention was also confirmed through multiple sustained rounds of tumor cell stimulation experiments antitumor, at the cytokine IL-7 concentrations of 0 ng/ml, 2.5 ng/ml, 5 ng/ml, 10 ng/ml, 50 ng/ml, and the IL-21 concentrations of 0 ng/ml, 12.5 ng/ml, 25 ng/ml, 50 ng/ml, 250 ng/ml. The T cells were activated with 50 ng/ml anti-CD3 antibody (Recombinant anti-Human CD3 mAb, GMP-A018) for 12-24 h, followed by transduction with CD 19-targeting lentiviral vector, and culture in the system containing both IL-7 and IL-21 cytokines at different concentrations. The cell harvest was completed 14-28 hours after the viral transduction. CD19-targeting CAR-T cells (CD19-targeting CAR-T cells as in patent 201710613317.6) prepared using the above solution were co-cultured with Nalm6-Luc-GFP tumor cells at the effector-to-target ratio of 1:2, and subjected to multiple repeated tumor killing assays. The proportion of GFP+ tumor cells in the cell population was detected by flow cytometry. When the proportion of the GFP+ cells was below 10%, the cells were collected and counted, and the tumor cells were replenished again at the effector-to-target ratio of 1:2 to perform repeated stimulation and killing. The point at which the proportion of the GFP+ tumor cells in the cell population exceeded 90% was taken as an endpoint of the killing assay. The number of rounds of tumor cell stimulation and the proliferation fold of CAR-T cells during the stimulation process in each test group were calculated. The functionality and persistence of the CAR-T cells under different preparation solutions were reflected by the number of rounds of target cell stimulation on the CAR-T cells and proliferative capacity after the stimulation. The results are as shown in FIG. 9B, and only with the cytokine combination of 2.5-10 ng/ml IL-7 and 12.5-50 ng/ml IL-21 did the CAR-T cells prepared by the preparation solution of the present invention demonstrate favorable persistence and proliferative potential.

The P-CAR solution of the present invention employed the anti-CD3 antibody to activate the T cells, and on this basis, the cytokine combination used thereby can also be IL-2 alone (solution 2 of Example 3); or IL-7 alone, that is, a combination with IL-21 at the concentration of 0 ng/ml; or IL-21 alone, that is, a combination with IL-7 at the concentration of 0 ng/ml. In order to validate the feasibility and advantage of the above combinations, and confirm the concentration ranges of different cytokine components, a validation experiment of killing and cytokine secretion was conducted on the P-CAR short-duration preparation solution in which the T cells were activated using 50 ng/ml anti-CD3 antibody (Recombinant anti-Human CD3 mAb, GMP-A018) and the prior art CD3CD28 + IL-2 short-duration preparation solution (see Example 4 for specific solution).

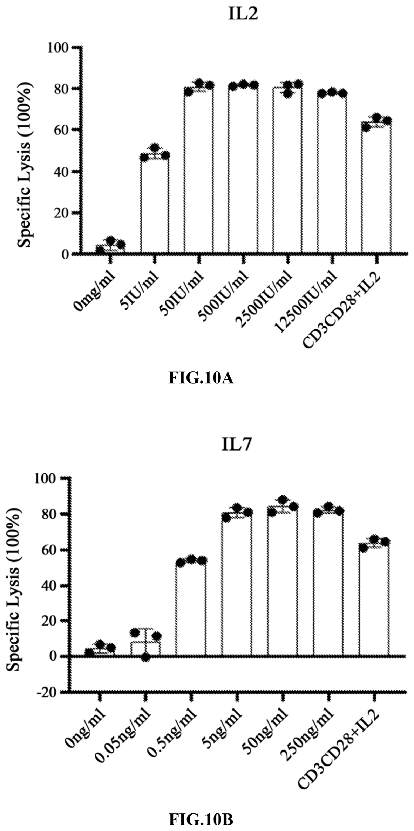

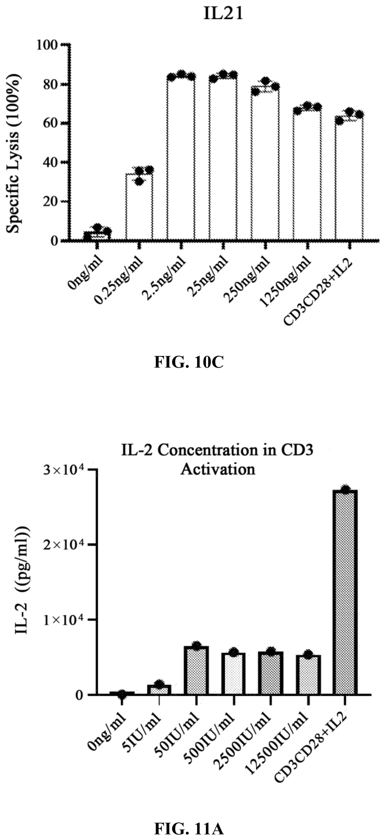

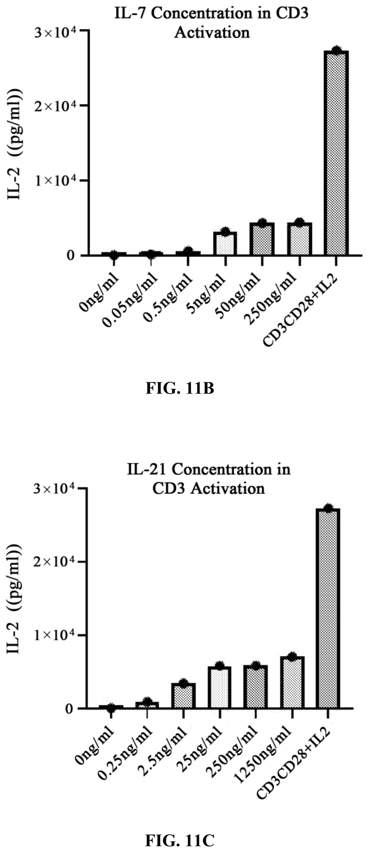

The results are as shown in FIGS. 10A-10C, Table 12, FIGS. 11A-11C, and Table 13, wherein FIG. 10A shows validation of killing with IL-2 as the sole cytokine component, FIG. 10B shows validation of killing with IL-7 as the sole cytokine component, and FIG. 10C shows validation of killing with IL-21 as the sole cytokine component, FIG. 11A shows IL-2 secretion validation with IL-2 as the sole cytokine component, FIG. 11B shows IL-2 secretion validation with IL-7 as the sole cytokine component, and FIG. 11C shows IL-2 secretion validation with IL-21 as the sole cytokine component.

**Table 12 Killing Percentage of P-CAR Process with Different Cytokine Components**

| IL2 | | | | | | CD3CD28 |
|---|---|---|---|---|---|---|
| 0 ng/ml | 5 IU/ml | 50 IU/ml | 500 IU/ml | 2500 IU/ml | 12500 IU/ml | IL-2 100 IU/ml |
| 6.8 | 51.62 | 81.73 | 82.16 | 81.7 | 78.43 | 66.05 |
| 1.83 | 47.87 | 82.59 | 81.15 | 77.64 | 77.74 | 64.63 |
| 4.89 | 46.83 | 78.48 | 81.83 | 82.22 | 77.6 | 61.29 |

| IL-7 | | | | | | CD3CD28 |
|---|---|---|---|---|---|---|
| 0 ng/ml | 0.05 ng/ml | 0.5 ng/ml | 5 ng/ml | 50 ng/ml | 250 ng/ml | IL-2 100 IU/ml |
| 6.8 | 13.36 | 54.17 | 83.75 | 88.21 | 80.81 | 66.05 |
| 1.83 | -0.31 | 52.81 | 81.27 | 84.48 | 84.4 | 64.63 |
| 4.89 | 11.45 | 54.88 | 78.16 | 81.24 | 82.1 | 61.29 |

| IL21 | | | | | | CD3CD28 |
|---|---|---|---|---|---|---|
| 0 ng/ml | 0.25 ng/ml | 2.5 ng/ml | 25 ng/ml | 250 ng/ml | 1250 ng/ml | IL-2 100 IU/ml |
| 6.8 | 30.38 | 83.92 | 84.77 | 78.85 | 68.39 | 66.05 |
| 1.83 | 35.69 | 85.27 | 85.32 | 81.69 | 69.12 | 64.63 |
| 4.89 | 36.31 | 83.62 | 82.89 | 76.18 | 66.35 | 61.29 |

**Table 13 IL-2 Cytokine Secretion of P-CAR Process with Different Cytokine Components after CAR-T Cell Activation, pg/ml**

| IL2 | | | | | | CD3CD28 |
|---|---|---|---|---|---|---|
| 0 ng/ml | 5 IU/ml | 50 IU/ml | 500 IU/ml | 2500 IU/ml | 12500 IU/ml | IL-2 100 IU/ml |
| 101.35 | 1473.867 | 6596.637 | 5740.811 | 5829.036 | 5428.322 | 27390.291 |

| IL-7 | | | | | | CD3CD28 |
|---|---|---|---|---|---|---|
| 0 ng/ml | 0.05 ng/ml | 0.5 ng/ml | 5 ng/ml | 50 ng/ml | 250 ng/ml | IL-2 100 IU/ml |
| 101.35 | 208.22 | 660.818 | 3225.217 | 4381.076 | 4465.877 | 27390.291 |

| IL21 | | | | | | CD3CD28 |
|---|---|---|---|---|---|---|
| 0 ng/ml | 0.25 ng/ml | 2.5 ng/ml | 25 ng/ml | 250 ng/ml | 1250 ng/ml | IL-2 100 IU/ml |
| 101.35 | 994.967 | 3498.233 | 5888.864 | 5920.49 | 7100.453 | 27390.291 |

In the above tables, entries marked solely with IL-2, IL-7, or IL-21 refer to preparation of the CAR-T cells using the P-CAR preparation process, and the cytokine component in the process was IL2, IL-7, or IL21, respectively.

The results demonstrate that, on the basis of using the anti-CD3 antibody activation in the P-CAR solution of the present invention, the cytokine combination used therein can also be IL-2 alone in the concentration range of 5-12,500 IU/ml (solution 2 of Example 3); IL-7 alone, i.e., a combination of IL-7 in the concentration range of 0.5-250 ng/ml and IL-21 at the concentration of 0 ng/ml; or IL-21 alone, i.e., a combination of IL-21 in the concentration range of 0.25-1,250 ng/ml and IL-7 at the concentration of 0 ng/ml. The CAR-T cells prepared using the P-CAR process with the above combinations had excellent killing activity against tumors, being safer than those by the existing CD3/CD28 process.

Amino acid sequences of CAR used in the above examples of the present invention are shown in the following Sequence Listing. It should be understood that the following sequences are merely exemplary sequences of the examples of the present invention, and are not intended to limit the solutions of the present invention in any way.

### Sequence Listing:

| SEQ ID NO. | Element | SEQ |
|---|---|---|
| 1 | CD19C AR | DIQMTQSPSSLSASVGDRVTITCRASQDISKYLNWYQQKPGKAPR LLIYHTSRLHSGVPSRFSGSGSGTDYTLTISSLQPEDFATYYCQQG NTLPYTFGGGTRLEIKGSTSGSGKPGSGEGSTKGQVQLQESGPGL VKPSQTLSLTCTVSGVSLPDYGVSWIRQPPGKALEWLGVIWGSET TYYSTSLKTRLTISKDNSKNQVVLTMTNMDPVDTATYYCAKHYY YGGSYAMDYWGQGSSVTVSSTTTPAPRPPTPAPTIASQPLSLRPEA CRPAAGGAVHTRGLDFACDIYIWAPLAGTCGVLLLSLVITLYCKR GRKKLLYIFKQPFMRPVQTTQEEDGCSCRFPEEEEGGCELRVKFS RSADAPAYKQGQNQLYNELNLGRREEYDVLDKRRGRDPEMGGK PRRKNPQEGLYNELQKDKMAEAYSEIGMKGERRRGKGHDGLYQ GLSTATKDTYDALHMQALPPR |
| 2 | CD19/C D22-CAR | DIQMTQSPSSLSASVGDRVTITCRASQDISKYLNWYQQKPGKAPR LLIYHTSRLHSGVPSRFSGSGSGTDYTLTISSLQPEDFATYYCQQG NTLPYTFGGGTRLEIKGSTSGSGKPGSGEGSTKGQVQLQESGPGL VKPSQTLSLTCTVSGVSLPDYGVSWIRQPPGKALEWLGVIWGSET TYYSTSLKTRLTISKDNSKNQVVLTMTNMDPVDTATYYCAKHYY YGGSYAMDYWGQGSSVTVSSTTTPAPRPPTPAPTIASQPLSLRPEA CRPAAGGAVHTRGLDFACDIYIWAPLAGTCGVLLLSLVITLYCKR GRKKLLYIFKQPFMRPVQTTQEEDGCSCRFPEEEEGGCELRVKFS RSADAPAYKQGQNQLYNELNLGRREEYDVLDKRRGRDPEMGGK PRRKNPQEGLYNELQKDKMAEAYSEIGMKGERRRGKGHDGLYQ GLSTATKDTYDALHMQALPPRGSGATNFSLLKQAGDVEENPGPE TTLTQSPATLSVSPGERATLSCRASQSVSSNLAWYQQKPGQAPRL LIYGASTRATGIPARFSGSGSGTEFTLTISSLQSEDFAIYYCQQYHS WPPLTFGGGTKLEIKRGSTSGSGKPGSGEGSTKGQVQLVQSGAEV KKPGASVKVSCKASGYTFTSYGISWVRQAPGQGLEWVGWISAY NGNTIYAQKLQGRVTMTTDTSTSTAYMELRSLRSDDTAVYYCAR DFQGIAVADLDYWGQGTLVTVSSKPTTTPAPRPPTPAPTIASQPLS LRPEACRPAAGGAVHTRGLDFACDFWVLVVVGGVLACYSLLVT VAFIIFWVRSKRSRGGHSDYMNMTPRRPGPTRKHYQPYAPPRDF AAYRSRVKFSRSADAPAYKQGQNQLYNELNLGRREEYDVLDKRR |
| | | GRDPEMGGKPRRKNPQEGLYNELQKDKMAEAYSEIGMKGERRR GKGHDGLYQGLSTATKDTYDALHMQALPPR |
| 3 | CEA-CAR | DIQMTQSPSSLSASVGDRVTITCSTSSSVSYMHWYQQKPGKAPRL LIYSTSNLASGVPSRFSGSGSGTDFTFTISSLQPEDIATYYCHQWSS YPTFGQGTKVEIKGSTSGSGKPGSGEGSTKGQVQLQESGPGLVRP SQTLSLTCTVSGFTISSGYSWHWVRQPPGRGLEWIGYIQYSGITN YNPSLKSRVTMLVDTSKNQFSLRLSSVTAADTAVYYCAREDYDY HWYFDVWGQGSTVTVSSTTTPAPRPPTPAPTIASQPLSLRPEACRP AAGGAVHTRGLDFACDIYIWAPLAGTCGVLLLSLVITLYCKRGRK KLLYIFKQPFMRPVQTTQEEDGCSCRFPEEEEGGCELRVKFSRSA DAPAYKQGQNQLYNELNLGRREEYDVLDKRRGRDPEMGGKPRR KNPQEGLYNELQKDKMAEAYSEIGMKGERRRGKGHDGLYQGLS TATKDTYDALHMQALPPR |

## Claims

1. A method of preparing a cell population expressing a chimeric antigen receptor (CAR), **characterized by** comprising:
step (i): contacting the cell population with a CD3/TCR complex agonist;
step (ii): contacting the cell population with a nucleic acid molecule encoding the CAR, thereby providing cells containing the nucleic acid molecule, and
step (iii): culturing the cell population for a period of time, and harvesting the cell population.

2. The method according to claim 1, **characterized in that** the cell population is not in contact with a CD28 agonist in step (i), steps (i) and (ii), steps (i), (ii), and (iii), or throughout an entire preparation process.

3. The method according to claim 1 or 2, **characterized in that** the CD3/TCR complex agonist comprises an anti-CD3 antibody.

4. The method according to any one of claims 1-3, **characterized in that** the cell population is in contact with an immune cell-activating cytokine in step i), steps i) and ii), steps i), ii), and iii), or throughout an entire preparation process, wherein the immune cell-activating cytokine excludes IL-2.

5. The method according to claim 4, **characterized in that** the immune cell-activating cytokine comprises one or more selected from the group consisting of IL-7, IL-15, IL-21, IL-12, IL-18, and IL-6.

6. The method according to claim 5, **characterized in that** the cytokine comprises a combination of IL-7 and IL-21.

7. The method according to claim 5 or 6, **characterized in that** a concentration of the IL-7 when in contact with the cell population is 2.5-10 ng/ml.

8. **The method** according to any one of claims 5-7, **characterized in that** a concentration of the IL-21 when in contact with the cell population is 12.5-50 ng/ml.

9. The method according to claim 3, **characterized in that** a concentration of the anti-CD3 antibody in contact with the cell population in step i) is ≥ 25 ng/ml, for example, 25-200 ng/ml.

10. The method according to any one of claims 1-3, **characterized in that** the cell population is in contact with an immune cell-activating cytokine in step i), steps i) and ii), steps i), ii), and iii), or throughout an entire preparation process, wherein the immune cell-activating cytokine comprises IL-2.

11. The method according to claim 10, **characterized in that** a concentration of the IL-2 is 100 IU/ml-500 IU/ml, 150 IU/ml-450 IU/ml, 200 IU/ml-400 IU/ml, or 250 IU/ml-350 IU/ml.

12. The method according to any one of claims 1-11, **characterized in that** a duration from start of step ii) to harvest of the cell population is ≤ 72 hours or ≤ 48 hours.

13. The he method according to any one of claims 1-12, **characterized in that** a duration from start of step ii) to harvest of the cell population is ≥ 6 hours.

14. The method according to any one of claims 1-13, **characterized in that** a duration from step i) until start of step ii) is 12-24 hours, and a duration from the start of step ii) to harvest of the cell population is 14-28 hours.

15. The method according to any one of claims 1-14, **characterized in that** a duration from start of step i) to harvest of the cell population is 1 to 4 days.

16. The method according to any one of claims 1-15, **characterized in that** a duration from start of step i) to harvest of the cell population is 1 to 2 days.

17. The method according to any one of claims 1-16, **characterized in that** the cell population comprises T cells.

18. The method according to claim 17, **characterized in that** the T cells are derived from peripheral blood mononuclear cells (PBMCs).

19. A composition for preparing a cell population expressing a chimeric antigen receptor (CAR), **characterized by** comprising a CD3/TCR complex agonist and an immune cell-activating cytokine, wherein the immune cell-activating cytokine excludes or comprises IL-2.

20. The composition according to claim 19, **characterized in that** the CD3/TCR complex agonist comprises an anti-CD3 antibody.

21. The composition according to claim 20, **characterized in that** a concentration of the anti-CD3 antibody is ≥ 25 ng/ml, for example, 25-200 ng/ml.

22. The composition according to any one of claims 19-21, **characterized in that** the immune cell-activating cytokine comprises one or more selected from the group consisting of IL-7, IL-15, IL-21, IL-12, IL-18, and IL-6.

23. The composition according to claim 22, **characterized in that** the immune cell-activating cytokine comprises a combination of IL7 and IL21.

24. The composition according to claim 23, **characterized in that** a concentration of the IL-7 is 2.5-10 ng/ml.

25. The composition according to claim 23 or 24, **characterized in that** a concentration of the IL-21 is 12.5-50 ng/ml.

26. The composition according to any one of claims 19 to 25, excluding a CD28 agonist.

27. A kit, comprising the composition according to any one of claims 19-26, and excluding IL-2 and CD28 agonist.

28. Use of the composition according to any one of claims 19-26, or the kit according to claim 27 for preparing a cell population expressing a chimeric antigen receptor (CAR).

29. The use according to claim 26, **characterized in that** the cell population comprises T cells.

30. A cell culture medium for preparing a cell population expressing a chimeric antigen receptor (CAR), comprising a CD3/TCR complex agonist and an immune cell-activating cytokine, **characterized in that** the immune cell-activating cytokine excludes IL-2.

31. The culture medium according to claim 28, **characterized in that** the CD3/TCR complex agonist comprises an anti-CD3 antibody.
